# EUROPÄISCHE PATENTSCHRIFT

(11) **EP 1 581 490 B1**
(45) Veröffentlichungstag und Bekanntmachung des Hinweises auf die Patenterteilung: **26.01.2011**
(21) Anmeldenummer: 03785875.0
(22) Anmeldetag: 18.12.2003
(51) Int. Cl.: C07D 207/40, A61K 31/40, A61P 25/04

(54) **SUBSTITUIERTE 5-AMINOMETHYL-1H-PYRROL-2-CARBONS UREAMIDE**
SUBSTITUTED 5-AMINOMETHYL-1H-PYRROLE-2-CARBOXAMIDES
AMIDES D'ACIDE 5-AMINOMETHYL-1H-PYRROL-2-CARBOXYLIQUE SUBSTITUES

(30) Priorität: 20.12.2002 DE 10261131
(43) Veröffentlichungstag der Anmeldung: 05.10.2005
(73) Patentinhaber: Grünenthal GmbH, 52078 Aachen (DE)
(72) Erfinder: MERLA, Beatrix, 52078 Aachen (DE); SUNDERMANN, Corinna, 52074 Aachen (DE); JAGUSCH, Utz-Peter, 52066 Aachen (DE); ENGLBERGER, Werner, 52223 Stolberg (DE); HENNIES, Hagen-Heinrich, 52152 Simmerath (DE); KÖGEL, Babette-Yvonne, 52379 Langerwehe-Hamich (DE)
(74) Vertreter: Brosch, Oliver
(86) Internationale Anmeldenummer: PCT/EP2003/014496
(87) Internationale Veröffentlichungsnummer: WO 2004/058707

(56) Entgegenhaltungen:
- TUSHAR CHAKRABORTY ET AL: "Development of 5-(aminomethyl)pyrrole-2-carboxylic acid as a constrained surrogate of Gly-Alanine and its application in peptidomimetic studies" TETRAHEDRON LETTERS, Bd. 43, 5. Dezember 2001 (2001-12-05) - 12. Februar 2002 (2002-02-12), Seiten 2589-2592, XP002276009
- SCOTT, MALCOLM ET AL: "Piperazinylalkyl heterocycles as potential antipsychotic agents" J.MED.CHEM., Bd. 38, Nr. 21, 1995, Seiten 4198-4210, XP002276010

## Beschreibung

Die vorliegende Erfindung betrifft substituierte 5-Aminomethyl-1H-pyrrol-2-carbonsäureamide, Verfahren zu deren Herstellung, Arzneimittel enthaltend diese Verbindungen sowie die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie beispielsweise das Morphin, sind bei der Therapie starker bis sehr starker Schmerzen wirksam, weisen jedoch unerwünschte Begleiterscheinungen, wie beispielsweise Atemdepression, Erbrechen, Sedierung oder Obstipation, auf. Nach weiteren schmerzhemmenden Mitteln wird weltweit geforscht.

Aufgabe der vorliegenden Erfindung war es daher, neue Wirkstoffe zur Verfügung zu stellen, die sich insbesondere zur Anwendung in Arzneimitteln, vorzugsweise in Arzneimitteln zur Bekämpfung von Schmerzen eignen.

Diese Aufgabe wurde durch die erfindungsgemäßen substituierten 5-Aminomethyl-1H-pyrrol-2-carbonsäureamide der nachstehenden allgemeinen Formel I gelöst.

Es wurde überraschenderweise gefunden, daß die erfindungsgemäßen substituierten 5-Aminomethyl-1H-pyrrol-2-carbonsäureamide der nachstehenden allgemeinen Formel I eine hohe Affinität für den ORL ("opioid receptor like")-1-Rezeptor wie auch für den µ-Opioid-Rezeptor aufweisen und sich daher zur Regulation dieser Rezeptoren eignen. Ferner führen die erfindungsgemäßen substituierten 5-Aminomethyl-1H-pyrrol-2-carbonsäureamide der nachstehenden allgemeinen Formel I zur Inhibierung des Noradrenalin-Uptakes sowie zur Inhibierung des 5-Hydroxytryptamin-(5-HT)-Uptakes.

Die erfindungsgemäßen substituierten 5-Aminomethyl-1H-pyrrol-2-carbonsäureamide der nachstehenden allgemeinen Formel I zeigen insbesondere eine ausgeprägte Wirksamkeit bei der Bekämpfung von Schmerzen, vorzugsweise chronischen Schmerzen und/oder akuten Schmerzen und/oder neuropathischen Schmerzen. Des weiteren eignen sich die erfindungsgemäßen substituierten 5-Aminomethyl-1H-pyrrol-2-carbonsäureamide auch zur Behandlung von Entzugserscheinungen, Gedächtnis-Störungen, neurodegenerativen Erkrankungen, vorzugsweise Morbus Parkinson, Morbus Huntington oder Morbus Alzheimer, Epilepsie, Störungen des cardiovaskulären Systems, Wasserspeicher-Krankheiten, intestinaler Motilität (Diarrhoe), Harninkontinenz, Anorexie, Pruritus, Depressionen, Tinnitus, sexueller Dysfunktionen, vorzugsweise erektiler Dysfunktionen, Atemwegserkrankungen, oder zur Diurese, zur Beeinflussung des cardiovaskulären Systems, vorzugsweise zur Vasodilatation der Arterien, zur Unterdrückung des Miktionsreflexes, zur Anxiolyse, zur Regulation des Elektrolyt-Haushaltes, zur Regulation, vorzugsweise Stimulation, der Nahrungsaufnahme, zur Reduzierung des Suchtpotentials von Opioiden, insbesondere von Morphin, zur Modulation der Bewegungsaktivität, zur Beeinflussung der Wirkung von µ-Agonisten, insbesondere Morphin.

Ein Gegenstand der vorliegenden Erfindung sind daher substituierte 5-Aminomethyl-1H-pyrrol-2-carbonsäureamide der folgenden allgemeinen Formel I, 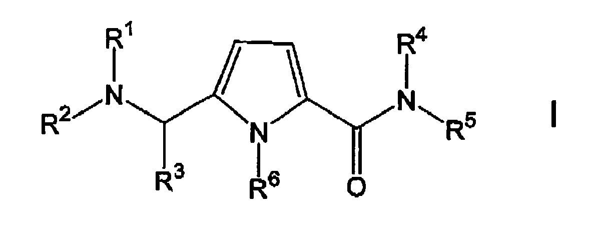 worin
R¹ für Wasserstoff, einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroaryl-Rest oder für einen über eine ggf. substituierte C₁₋₃-Alkylen-Gruppe gebundenen, ggf. wenigstens einfach substituierten, fünf- oder sechsgliedrigen Aryl- oder Heteroaryl-Rest steht,
R² für einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroaryl-Rest oder für einen über eine ggf. substituierte C₁₋₃-Alkylen-Gruppe gebundenen, ggf. wenigstens einfach substituierten, fünf- oder sechsgliedrigen Aryl- oder Heteroaryl-Rest steht, oder
R¹ und R² gemeinsam mit dem sie verbindenden Stickstoffatom als Ringglied einen gesättigten oder ungesättigten, ggf. wenigstens einfach substituierten, fünf- oder sechsgliedrigen, cycloaliphatischen Rest bilden, der ggf. wenigstens ein weiteres Heteroatom ausgewählt aus der Gruppe bestehend aus N, O und S als Ringglied aufweist,
R³ für einen ggf. wenigstens einfach substituierten Phenylrest oder eine Alkylester-Gruppe mit C₁₋₃ im Alkylteil steht,
R⁴ und R⁵, gleich oder verschieden, für Wasserstoff, einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten fünf- oder sechsgliedrigen Aryl- oder Heteroaryl-Rest oder für einen über eine ggf. substituierte C₁₋₃-Alkylen-Gruppe gebundenen, ggf. wenigstens einfach substituierten, fünf- oder sechsgliedrigen Aryl- oder Heteroaryl-Rest stehen, oder
R⁴ und R⁵ gemeinsam mit dem sie verbindenden Stickstoffatom als Ringglied einen gesättigten oder ungesättigten, ggf. wenigstens einfach substituierten fünf-, sechs- oder sechsgliedrigen cycloaliphatischen Rest bilden, der ggf. wenigstens ein weiteres Heteroatom ausgewählt aus der Gruppe bestehend aus N, O und S als Ringglied aufweist, und
R⁶ für einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroaryl-Rest, einen über eine ggf. wenigstens einfach substituierte C₁₋₃-Alkylen-Gruppe gebundenen, ggf. wenigstens einfach substituierten fünf- oder sechsgliedrigen Aryl- oder Heteroaryl-Rest steht, gegebenenfalls in Form ihrer reinen Stereolsomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder ihrer Basen oder in Form ihrer Salze, Insbesondere der physiologisch verträglichen Salze, oder jeweils in Form ihrer Solvate, insbesondere der Hydrate.

Bevorzugt sind erfindungsgemäße substituierte 5-Aminomethyl-1H-pyrrol-2-carbonsäureamide der allgemeinen Formel I, in denen R¹ für einen linearen oder verzweigten, gesättigten aliphatischen C₁₋₃-Rest, besonders bevorzugt für einen Methyl-Rest steht und die übrigen Reste R²-R⁶ die vorstehend genannte Bedeutung haben, gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotameren, in einem bellebigen Mischungsverhältnis, oder jeweils in Form Ihrer Säuren oder ihrer Basen oder in Form ihrer Salze, insbesondere der physiologisch verträglichen Salze, oder jeweils in Form ihrer Solvate, insbesondere der Hydrate.

Ebenfalls bevorzugt sind erflndungsgemäße substituierte 5-Aminomethyl-1H-pyrrol-2-carbonsäureamide der allgemeinen Formel I, worin R² für einen linearen oder verzweigten, gesättigten aliphatischen C₁₋₃-Rest, besonders bevorzugt für einen Methyl-Rest steht und die übrigen Reste R¹ und R³ bis R⁶ die vorstehend genannte Bedeutung haben, gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotamere, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Weiterhin bevorzugt sind erfindungsgemäße substituierte 5-Aminomethyl-1H-pyrrol-2-carbonsäureamide der allgemeinen Formel I, worin die Reste R¹ und R² gemeinsam mit dem sie verbindenden Stickstoffatom als Ringglied einen gesättigten, ggf. Sauerstoff als weiteres Ringglied aufweisenden, fünf- oder sechsgliedrigen cycloaliphatischen Rest bilden, besonders bevorzugt gemeinsam für einen (CH₂)₄-, (CH₂)₅- oder (CH₂)₂-O-(CH₂)₂-Rest stehen, der mit dem sie verbindendenden Stickstoffatom als Ringglied einen Heterocyclus bildet, und jeweils die übrigen Reste R³ bis R⁶ die vorstehend genannte Bedeutung haben, gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

Des weiteren sind erfindungsgemäße substituierte 5-Aminomethyl-1H-pyrrol-2-carbonsäureamide der allgemeinen Formel I bevorzugt, worin R³ für einen ggf. wenigstens einfach substituierten Phenylrest oder eine Alkylester-Gruppe mit C₁₋₂, im Alkyltell, steht und jeweils die Reste R¹, R² und R⁴ bis R⁶ die vorstehend genannte Bedeutung haben, gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder ihrer Basen oder in Form Ihrer Salze, insbesondere der physiologisch verträglichen Salze, oder jeweils in Form Ihrer Solvate, insbesondere der Hydrate.

Ferner sind erfindungsgemäße substituierte 5-Aminomethyl-1H-pyrrol-2-carbonsäureamide der allgemeinen Formel I bevorzugt, worin die Reste R⁴ und R⁵, gleich oder verschieden, für Wasserstoff, Methyl, Ethyl, n-Propyl, iso-Propyl, n-Butyl, Cyclopentyl, Cyclohexyl, Benzyl oder Phenethyl stehen, und jeweils die Reste R¹ bis R³ und R⁶ die vorstehend genannte Bedeutung haben, gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder Ihrer Basen oder in Form ihrer Salze, insbesondere der physiologisch verträglichen Salze, oder jeweils in Form Ihrer Solvate, insbesondere der Hydrate.

Ebenfalls bevorzugt sind erfindungsgemäße substituierte 5-Aminomethyl-1H-pyrrol-2-carbonsäureamide der allgemeinen Formel I, worin die Reste R⁴ und R⁵ gemeinsam mit dem sie verbindenden Stickstoffatom als Ringglied einen gesättigten, ggf. Sauerstoff als weiteres Ringglied aufweisenden, fünf- oder sechsgliedrigen cycloaliphatischen Rest bilden, besonders bevorzugt gemeinsam für einen (CH₂)₄-, (CH₂)₅- oder (CH₂)₂-O-(CH₂)₂-Rest stehen, der mit dem sie verbindenden Stickstoffatom als Ringglied einen Heterocyclus bildet, und jeweils die übrigen Reste R¹ bis R³ und R⁶ die vorstehend genannte Bedeutung haben, gegebenenfalls in Form ihrer reinen Stereolsomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder ihrer Basen oder in Form ihrer Salze, insbesondere der physiologisch verträglichen Salze, oder jeweils In Form ihrer Solvate, insbesondere der Hydrate.

Bevorzugt sind auch erfindungsgemäße substituierte 5-Aminomethyl-1H-pyrrol-2-carbonsäureamide der allgemeinen Formel I, worin R⁶ für einen linearen oder verzweigten, gesättigten aliphatischen C₁₋₃-Rest, besonders bevorzugt für einen Methylrest steht und die Reste R¹ bis R⁵ jeweils die vorstehend genannte Bedeutung haben, gegebenenfalls in Form ihrer reinen Stereoisomeren, inbesondere Enantiomeren oder Diastereomeren, Ihrer Racemate oder in Form von Mischungen der Stereoisomeren, insbesondere der Enantiomeren. Diastereomeren oder Rotameren, in einem beliebigen Mischungsverhältnis, oder jeweils in Form Ihrer Säuren oder ihrer Basen oder in Form ihrer Salze, Insbesondere der physiologisch verträglichen Salze, oder jeweils in Form ihrer Solvate, insbesondere der Hydrate.

Sofern einer der vorstehend genannten Reste R¹ bis R⁶ für einen aliphatischen oder cycloaliphatischen Rest steht, der einfach oder mehrfach substituiert ist, können die Substituenten ausgewählt werten aus der Gruppe bestehend aus Halogen, Hydroxy, CN, CF₃, CHF₂, CH₂F, C₁₋₆-Alkyl, C₁₋₆-Alkoxy und ggf. wenigstens einfach substituiertem Phenyl, bevorzugt aus der Gruppe bestehend aus F, Cl, Br und Hydroxy. Sofern der Phenylsubstituent selbst einfach oder mehrfach substituiert ist, können dessen Substituenten ausgewählt werden aus der Gruppe bestehend aus Hydroxy, CN, CF₃, CHF₂, CH₂F, C₁₋₆-Alkyl und C₁₋₆-Alkoxy.

Sofern die vorstehend genannten Reste R¹ bis R⁶ eine einfach oder mehrfach substituierte Alkylen-Gruppe aufweisen, können deren Substituenten ausgewählt werden aus der Gruppe bestehend aus Halogen, Hydroxy, CN, CF₃, CHF₂, CH₂F, C₁₋₆-Alkoxy und ggf. wenigstens einfach substituiertem Phenyl, bevorzugt aus der Gruppe bestehend aus F, Cl, Br und Hydroxy. Sofern der Phenylsubstituent selbst einfach oder mehrfach substituiert ist, können dessen Substituenten ausgewählt werden aus der Gruppe bestehend aus Hydroxy, CN, CF₃, CHF₂, CH₂F, C₁₋₆-Alkyl und C₁₋₆-Alkoxy.

Welst einer der vorstehend genannten Reste R¹ bis R⁶ einen einfach oder mehrfach substituierten Aryl- oder Heteroaryl-Rest auf, so können die entsprechenden Substituenten ausgewählt werden aus der Gruppe bestehend aus Halogen, Hydroxy, CN, CF₃, CHF₂, CH₂F, NO₂, NH₂, C₁₋₆-Alkyl, C₁₋₆-Alkoxy und ggf. wenigstens einfach substituiertem Phenyl, bevorzugt aus der Gruppe bestehend aus F, Cl, Br, Hydroxy, OCH₃ und CH₃. Sofern der Phenylsubstituent selbst einfach oder mehrfach substituiert ist, können dessen Substituenten ausgewählt werden aus der Gruppe bestehend aus Hydroxy, CN, CF₃, CHF₂, CH₂F, C₁₋₆-Alkyl und C₁₋₆-Alkoxy.

Sofern einer der vorstehend genannten Reste R¹ bis R⁶ für einen cycloallphatischen Rest mit wenigstens einem Heteroatom oder einen Heteroarylrest steht, können die Heteroatome, sofern nicht anders angegeben, ausgewählt werden aus der Gruppe bestehend aus Sauerstoff, Stickstoff und Schwefel.

Geeignete aliphatische Reste, die ggf. einfach oder mehrfach substituiert sind, können beispielsweise ausgewählt werden aus der Gruppe bestehend aus Methyl, Ethyl, n-Propyl, iso-Propyl, n-Butyl, sec-Butyl, tert-Butyl, n-Pentyl, iso-Pentyl, neoPentyl, n-Hexyl, 2-Hexyl, n-Heptyl, n-Octyl, Vinyl, Ethinyl, Propenyl, Propinyl, Butenyl, Butinyl, Pentenyl, Pentinyl, Hexenyl, Hexinyl, Heptyl, Heptinyl, Octenyl und Octyl.

Geeignete cycloaliphatischen Reste, die ggf. einfach oder mehrfach substituiert sind und/oder ggf. wenigstens ein Heteroatom aufweisen, können beispielsweise ausgewählt werden aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl und Morpholinyl.

Geeignete Alkylen-Gruppen, die ggf. einfach oder mehrfach substituiert sind, können beispielsweise ausgewählt werden aus der Gruppe bestehend aus Methylen -(CH₂)-, Ethylen -(CH₂)₂-, Propylen -(CH₂)₃-, Butylen -(CH₂)₄-, Pentylen -(CH₂)₅- und Hexylen -(CH₂)₆-, jeweils ggf. auch verzweigt.

Geeignete Aryl-Reste, die ggf. einfach oder mehrfach substituiert sind, sind beispielsweise Phenyl oder Naphthyl, vorzugsweise Phenyl.

Geeignete Heteroaryl-Reste, die ggf. einfach oder mehrfach substituiert sind, können beispielsweise ausgewählt werden aus der Gruppe bestehend aus Pyrrolyl, Furyl, Thienyl, Pyrazolyl, Imidazolyl, Pyridinyl, Pyridazinyl und Pyrimidinyl.

Ganz besonders bevorzugt sind erfindungsgemäße substituierte 5-Aminomethyl-1H-pyrrol-2-carbonsäureamide ausgewählt aus der Gruppe bestehend aus

5-[Dimethylamino-(4-fluoro-phenyl)-methyl]-1-methyl-1H-pyrrol-2-carbonsäurecyclohexylamid,

5-[Dimethylamino-(4-fluoro-phenyl)-methyl]-1-methyl-1H-pyrrol-2-carbonsäurebutylamid,

{5-[Dimethylamino-(4-fluoro-phenyl)-methyl]-1-methyl-1H-pyrrol-2-yl}-pyrrolidin-1-ylmethanon,

5-[Dimethylamino-(4-bromo-phenyl)-methyl]-1-methyl-1H-pyrrol-2-carbonsäurecyclohexylamid,

5-[Dimethylamino-(4-bromo-phenyl)-methyl]-1-methyl-1H-pyrrol-2-carbonsäurebutylamid,

{5-[(4-Bromo-phenyl)-dimethylamino-methyl]-1-methyl-1H-pyrrol-2-yl}-pyrrolidin-1-ylmethanon,

5-[(4-Bromo-phenyl)-dimethylamino-methyl]-1-methyl-1H-pyrrol-2-carbonsäurephenethyl-amid,

{5-[(4-Bromo-phenyl)-dimethylamino-methyl]-1-methyl-1H-pyrrol-2-yl}-morpholin-4-yl-methanon,

5-(Dimethylamino-phenyl-methyl)-1-methyl-1H-pyrrol-2-carbonsäure-cyclohexylamid,

5-(Dimethylamino-phenyl-methyl)-1-methyl-1H-pyrrol-2-carbonsäure-butylamid,

[5-(Dimethylamino-phenyl-methyl)-1-methyl-1H-pyrrol-2-yl]-pyrrolidin-1-yl-methanon,

(5-Butylcarbamoyl-1-methyl-1H-pyrrol-2-yl)-dimethylamino-essigsäureethylester,

(5-Cyclohexylcarbamoyl-1-methyl-1H-pyrrol-2-yl)-dimethylaminoessigsäureethylester,

(5-Butylcarbamoyl-1-methyl-1H-pyrrol-2-yl)-piperidin-1-yl-essigsäureethylester und

(5-Cyclohexylcarbamoyl-1-methyl-1H-pyrrol-2-yl)-piperidin-1-yl-essigsäureethylester,

gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder ihrer Basen oder in Form ihrer Salze, insbesondere der physiologisch verträglichen Salze, besonders bevorzugt der Hydrochloride, oder jeweils in Form ihrer Solvate, insbesondere der Hydrate.

Ein weiterer Gegenstand der vorliegenden Erfindung ist ein Verfahren zur Herstellung der erfindungsgemäßen substituierten 5-Aminomethyl-1H-pyrrol-2-carbonsäureamide der allgemeinen Formel I, gemäß dem ein substituiertes 1H-pyrrol-2-carbonsäureamid der allgemeinen Formel II 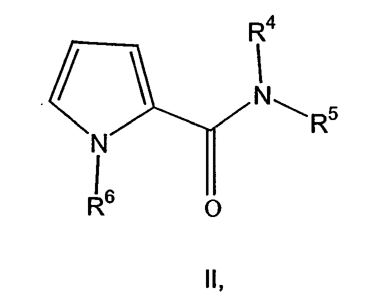 worin die Reste R⁴, R⁵ und R⁶ die vorstehend genannte Bedeutung haben, nach üblichen, dem Fachmann bekannten Methoden, vorzugsweise in einem geeigneten Lösungsmittel, wie z.B. CH₂Cl₂, CH₃CN, Dimethylformamid (DMF) oder Mischungen aus wenigstens zwei dieser Lösungsmittel, bei Raumtemperatur (ca. 20-25°C) mit einem Imminiumsalz der allgemeinen Formel III, 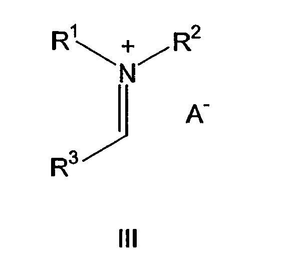 worin R¹ bis R³ die vorstehend genannte Bedeutung haben und A⁻ für ein geeignetes Anion, vorzugsweise für Cl⁻, AlCl₄⁻, Br⁻, I⁻ oder CF₃-SO₃⁻ (Triflat-Anion) steht, zu einem erfindungsgemäßen substituierten 5-Aminomethyl-1H-pyrrol-2-carbonsäureamid der allgemeinen Formel I umgesetzt und dieses ggf. nach üblichen, dem Fachmann bekannten Methoden, vorzugsweise durch Extraktion, gereinigt und ggf. isoliert wird.

Die Verbindungen der allgemeinen Formel II können nach üblichen, dem Fachmann bekannten Methoden, beispielsweise aus den am Markt käuflich erhältlichen Reagenzien der allgemeinen Formel IV 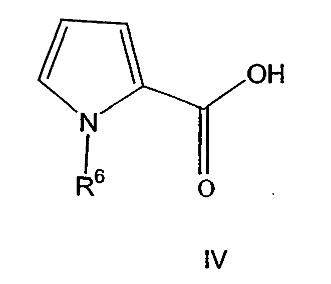 worin R⁶ die vorstehend genannte Bedeutung hat, hergestellt werden, wie z.B. in A.J. Carpenter, D.J. Chadwick, Journal of Organic Chemistry, 1985, 50, Seiten 4362-4368 beschrieben. Die entsprechende Literaturbeschreibung wird hiermit als Referenz eingeführt und gilt als Teil der Offenbarung.

Die Imminiumsalze der allgemeinen Formel III können ebenfalls nach üblichen, dem Fachmann bekannten Verfahren, beispielsweise aus den entsprechenden Aminalen der nachstehenden allgemeinen Formel V 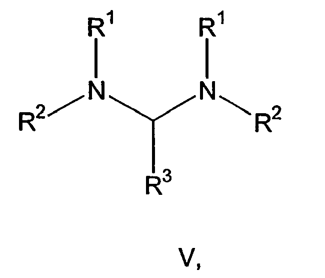 worin die Reste R¹, R² und R³ die vorstehend genannte Bedeutung haben, erhalten werden, wie beispielsweise in D. Seebach et al., Helv. Chim. Acta 1988, 71, Seiten 1999-2001 und N. Risch et al., Synthesis 1998, 11, Seiten 1609-1614 beschrieben. Die entsprechenden Literaturbeschreibungen werden hiermit als Referenz eingeführt und gelten als Teil der Offenbarung.

Auch die Herstellung der Aminale der allgemeinen Formel V kann nach literaturbekannten Methoden erfolgen, wie z.B. in D. Seebach et al., Helv. Chim. Acta 1988, 71, Seiten 1999-2001 und N. Risch et al., Synthesis 1998, 11, Seiten 1609-1614 beschrieben. Die entsprechenden Literaturbeschreibungen werden hiermit als Referenz eingeführt und gelten als Teil der Offenbarung.

Die erfindungsgemäßen substituierten 5-Aminomethyl-1H-pyrrol-2-carbonsäureamide der allgemeinen Formel I sowie entsprechende Stereoisomere können sowohl in Form ihrer freien Basen, ihrer freien Säuren wie auch in Form entsprechender Salze isoliert werden.

Die freien Basen der jeweiligen erfindungsgemäßen 5-Aminomethyl-1H-pyrrol-2-carbonsäureamide der allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden physiologisch verträglichen Salze überführt werden.

Die freien Basen der jeweiligen erfindungsgemäßen 5-Aminomethyl-1H-pyrrol-2-carbonsäureamide der allgemeinen Formel I sowie entsprechender Stereoisomere können bevorzugt durch Versetzen der in einem geeigneten organischen Lösungsmittel, wie z.B. Butan-2-on (Methylethylketon), gelösten Verbindungen der allgemeinen Formel I oder entsprechender Stereoisomere als freie Basen mit Trimethylsilylchlorid (TMSCI) in die entsprechenden Hydrochloride übergeführt werden.

Die freien Basen der jeweiligen 5-Aminomethyl-1H-pyrrol-2-carbonsäureamide der allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der jeweiligen 5-Aminomethyl-1H-pyrrol-2-carbonsäureamide der allgemeinen Formel I und entsprechender Stereoisomere durch Umsetzung mit einer geeigneten Base in die entsprechenden physiologisch verträglichen Salze überführt werden.

Die erfindungsgemäßen 5-Aminomethyl-1H-pyrrol-2-carbonsäureamide der allgemeinen Formel I und entsprechende Stereoisomere können ggf., ebenso wie die entsprechenden Säuren, die entsprechenden Basen oder Salze dieser Verbindungen, auch in Form ihrer Solvate, vorzugsweise ihrer Hydrate, erhalten werden.

Sofern die erfindungsgemäßen substituierten 5-Aminomethyl-1H-pyrrol-2-carbonsäureamide der allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 5-Aminomethyl-1H-pyrrol-2-carbonsäureamide der allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung sind daher Arzneimittel enthaltend wenigstens ein erfindungsgemäßes substituiertes 5-Aminomethyl-1H-pyrrol-2-carbonsäureamid der allgemeinen Formel I, gegebenenfalls in Form seines Racemates, seiner reinen Stereoisomeren, insbesondere Enantiomeren oder Diastereomeren, oder in Form von Mischungen der Stereoisomeren, insbesondere der Enantiomeren, Diastereomeren oder Rotameren, in einem beliebigen Mischungsverhältnis, oder jeweils in Form seiner Säuren oder seiner Basen oder in Form seiner Salze, insbesondere der physiologisch verträglichen Salze, besonders bevorzugt der Hydrochloride, oder jeweils in Form seiner Solvate, insbesondere der Hydrate, sowie ggf. physiologisch verträgliche Hilfsstoffe.

Diese Arzneimittel eignen sich insbesondere zur Regulation des ORL ("opioid receptor like")-1-Rezeptors, zur Regulation des µ-Opioid-Rezeptors, zur Inhibierung des Noradrenalin-Uptakes sowie zur Inhibierung des 5-Hydroxytryptamin-(5-HT)-Uptakes.

Ebenfalls bevorzugt eignen sich die erfindungsgemäßen Arzneimittel zur Bekämpfung von Schmerzen, vorzugsweise zur Bekämpfung von chronischen Schmerzen und/oder akuten Schmerzen und/oder neuropathischen Schmerzen.

Des weiteren eignen sich die erfindungsgemäßen Arzneimittel auch zur Behandlung von Entzugserscheinungen, Gedächtnis-Störungen, neurodegenerativen Erkrankungen, vorzugsweise Morbus Parkinson, Morbus Huntington oder Morbus Alzheimer, Epilepsie, Störungen des cardiovaskulären Systems, Wasserspeicher-Krankheiten, intestinaler Motilität (Diarrhoe), Harninkontinenz, Anorexie, Pruritus, Depressionen, Tinnitus, sexueller Dysfunktionen, vorzugsweise erektiler Dysfunktionen, Atemwegserkrankungen, oder zur Diurese, zur Beeinflussung des cardiovaskulären Systems, vorzugsweise zur Vasodilatation der Arterien, zur Unterdrückung des Miktionsreflexes, zur Anxiolyse, zur Regulation des Elektrolyt-Haushaltes, zur Regulation, vorzugsweise Stimulation, der Nahrungsaufnahme, zur Reduzierung des Suchtpotentials von Opioiden, insbesondere von Morphin, zur Modulation der Bewegungsaktivität, zur Beeinflussung der Wirkung von µ-Agonisten, insbesondere Morphin.

Ein weiterer Gegenstand der vorliegenden Erfindung ist die Verwendung eines oder mehrerer substituierter 5-Aminomethyl-1H-pyrrol-2-carbonsäureamide der allgemeinen Formel I, gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder ihrer Basen oder in Form ihrer Salze, insbesondere der physiologisch verträglichen Salze, besonders bevorzugt der Hydrochloride, oder jeweils in Form ihrer Solvate, insbesondere der Hydrate, zur Herstellung eines Arzneimittels zur Regulation des ORL ("opioid receptor like")-1-Rezeptors, zur Regulation des µ-Opioid-Rezeptors, zur Inhibierung des Noradrenalin-Uptakes oder zur Inhibierung des 5-Hydroxytryptamin-(5-HT)-Uptakes.

Ein weiterer Gegenstand der vorliegenden Erfindung ist die Verwendung eines oder mehrerer substituierter 5-Aminomethyl-1H-pyrrol-2-carbonsäureamide der allgemeinen Formel I, gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder ihrer Basen oder in Form ihrer Salze, insbesondere der physiologisch verträglichen Salze, besonders bevorzugt der Hydrochloride, oder jeweils in Form ihrer Solvate, insbesondere der Hydrate, zur Herstellung eines Arzneimittels zur Bekämpfung von Schmerzen, vorzugsweise chronischen Schmerzen und/oder akuten Schmerzen und/oder neuropathischen Schmerzen, zur Behandlung von Entzugserscheinungen, Gedächtnis-Störungen, neurodegenerativen Erkrankungen, vorzugsweise Morbus Parkinson, Morbus Huntington oder Morbus Alzheimer, Epilepsie, Störungen des cardiovaskulären Systems, Wasserspeicher-Krankheiten, intestinaler Motilität (Diarrhoe), Harninkontinenz, Anorexie, Pruritus, Depressionen, Tinnitus, sexueller Dysfunktionen, vorzugsweise erektiler Dysfunktionen, Atemwegserkrankungen, oder zur Diurese, zur Unterdrückung des Miktionsreflexes, zur Anxiolyse, zur Regulation des Elektrolyt-Haushaltes, zur Beeinflussung des cardiovaskulären Systems, vorzugsweise zur Vasodilatation der Arterien, zur Regulation, vorzugsweise Stimulation, der Nahrungsaufnahme, zur Reduzierung des Suchtpotentials von Opioiden, insbesondere von Morphin, zur Modulation der Bewegungsaktivität oder zur Beeinflussung der Wirkung von µ-Agonisten, insbesondere Morphin.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einem oder mehreren substituierten 5-Aminomethyl-1H-pyrrol-2-carbonsäureamiden der allgemeinen Formel I, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes, oder in Form ihres Solvates, insbesondere des Hydrates, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll.

Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Substituierte 5-Aminomethyl-1H-pyrrol-2-carbonsäureamide der allgemeinen Formel I, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes, oder in Form ihres Solvates, insbesondere des Hydrates, in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten 5-Aminomethyl-1H-pyrrol-2-carbonsäureamide der allgemeinen Formel I, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes, oder in Form ihres Solvates, insbesondere des Hydrates, auch verzögert freisetzen. Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge des jeweiligen substituierten 5-Aminomethyl-1H-pyrrol-2-carbonsäureamids der allgemeinen Formel I, gegebenenfalls in Form des Racemates, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form der Säure oder Base oder in Form des Salzes, insbesondere eines physiologisch verträglichen Salzes, oder jeweils in Form des Solvates, insbesondere des Hydrates, kann variieren und ist beispielsweise abhängig vom Gewicht oder Alter des Patienten sowie von der Applikationsart, der Indikation und dem Schweregrad der Erkrankung. Üblicherweise werden 0,005 bis 500 mg/kg, vorzugsweise 0,05 bis 5 mg/kg Körpergewicht des Patienten wenigstens eines substituierten 5-Aminomethyl-1H-pyrrol-2-carbonsäureamids der allgemeinen Formel I, gegebenenfalls in Form seines Racemates, seines reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form seiner Säure oder seiner Base oder in Form seines Salzes, insbesondere eines physiologisch verträglichen Salzes, oder in Form ihres Solvates, insbesondere des Hydrates, appliziert.

### Pharmakologische Methoden:

### a) Methode zur Bestimmung der Affinität zu dem ORL-1-Rezeptor

Die Affinität des jeweiligen substituierten 5-Aminomethyl-1H-pyrrol-2-carbonsäureamids der allgemeinen Formel I zu dem ORL-1-Rezeptor wurde in einem Rezeptorbindungsassay mit ³H-Nociceptin/Orphanin FQ mit Membranen von rekombinanten CHO-ORL1 Zellen bestimmt, wie von Ardati et al. in Mol. Pharmacol., 51, 1997, Seiten 816-824 beschrieben. Die entsprechende Literaturbeschreibung wird hiermit als Referenz eingeführt und gilt als Teil der Offenbarung.

Die Konzentration von ³H-Nociceptin/Orphanin FQ betrug bei diesen Versuchen 0,5 nM. Die Bindungsassays wurden mit jeweils 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg, Deutschland), durch einstündige Inkubation des Ansatzes bei Raumtemperatur (ca. 20-25 °C) und anschließende Messung im Szintillationscounter Trilux (Wallac, Finnland), bestimmt.

### b) Methode zur Bestimmung der Affinität zum humanen µ-Opiatrezeptor

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten 5-Aminomethyl-1H-pyrrol-2-carbonsäureamids der allgemeinen Formel I mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der zu prüfenden Verbindungen von 1 µmol/l bestimmt und als Prozent Hemmung der spezifischen Bindung angegeben. Ausgehend von der prozentualen Verdrängung durch unterschiedliche Konzentrationen der zu prüfenden Verbindungen der allgemeinen Formel I wurden IC₅₀ Hemmkonzentrationen berechnet, die eine 50-prozentige Verdrängung des radioaktiven Liganden bewirken. Durch Umrechnung mittels der Cheng-Prusoff-Beziehung wurden Kᵢ-Werte für die Prüfsubstanzen erhalten.

### c) Methode zur Bestimmung der Noradrenalin- und der 5HT-Uptake-Inhibierung:

Für in vitro Studien wurden Synaptosomen aus Rattenhirnarealen (verwendet wurde die sogenannte "P2"-Fraktion) frisch isoliert, wie in der Veröffentlichung "The isolation of nerve endings from brain" von E.G. Gray und V.P. Whittaker, J. Anatomy 76, Seiten 79-88, 1962, beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wurde in eisgekühlter 0,32 M Sucrose (100 mg Gewebe / 1 mL) in einem Glas-Homogenisierer mit Teflonstößel homogenisiert, indem fünf volle Auf-und Abschläge bei 840 Umdrehungen /Minute benutzt wurden.

Das Homogenat wurde bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mU 100 mg des ursprünglichen Gewichts) noch einmal suspendiert wurden.

Der jeweilige Uptake wurde in einer 96-well Mikrotiterplatte gemessen. Das Volumen betrug 250 µl und die Inkubation erfolgte bei Raumtemperatur (ca. 20-25 °C) unter O₂ Atmosphäre.

Die Inkubationszeit betrug 7,5 Minuten für [³H]-NA und 5 Minuten für [³H]-5-HT. Anschließend wurden die 96 Proben durch eine Unifilter GF/B^{®} Mikrotiterplatte (Packard) filtriert und mit 200 mL inkubierten Puffer mit Hilfe eines "Brabdel Cell-Harvester MPXRI-96T" gewaschen. Die Unifilter GF/B Platte wurde bein 55°C 1 Stunde getrocknet. Im Anschluß wurde die Platte mit einem Back seal^{®} (Packard) verschlossen und 35 µl Szintilationsflüssigkeit pro Well (Ultima Gold^{®}, Packard) versetzt. Nach dem Verschließen mit einem top seal^{®} (Packard) wurde, nach der Einstellung des Gleichgewichts (etwa 5 Stunden), die Radioaktivität in einem "Trilux 1450 Microbeta" (Wallac) bestimmt.

Folgende Kenndaten wurden für den NA-Transporter ermittelt:
NA-Uptake : Km = 0,32 ± 0,11 µM

Die Menge des bei der vorstehenden Bestimmung eingesetzten Proteins entsprach den aus der Literatur bekannten Werten, wie z.B in "Protein measurement with the folin phenol reagent", Lowry et al., J. Biol. Chem., 193, 265-275, 1951 beschrieben. Eine detaillierte Methodenbeschreibung kann auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden. Die entsprechenden Literaturbeschreibungen werden hiermit als Referenz eingeführt und gelten als Teil der Offenbarung.

### d) Untersuchung auf analgetische Wirksamkeit im Writhing-Test

Die Untersuchung der erfindungsgemäßen Verbindungen der allgemeinen Formel I auf analgetische Wirksamkeit wurde im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Verbindungsdosis erhielten 10 Minuten nach intravenöser Gabe der zu untersuchenden Verbindungen 0,3 ml/Maus einer 0,02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere wurden einzeln in Beobachtungskäfige gesetzt. Mit Hilfe eines Drucktastenzählers wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten hatten. Alle Verbindungen wurden in der Standarddosierung von 10 mg/kg getestet.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die Messung der NMR-Spektren erfolgte auf einem Gerät vom Typ Bruker DPX 300 für die 300 MHz-Spektren und auf einem Gerät vom Typ Bruker DRX 600 für die 600 MHz-Spektren.

Die jeweiligen Chemikalien und Lösungsmittel wurden kommerziell von den üblichen Herstellern erworben.

### A)

### Allgemeine Synthesevorschrift zur Herstellung von Verbindungen der allgemeinen Formel II:

Die jeweilige Pyrrol-2-carbonsäure (50 mmol) der allgemeinen Formel IV wurde in 200 ml Toluol suspendiert. Anschließend wurden unter Kühlung im Eisbad langsam 100 mmol Natriumhydrid (NaH) zugegeben und für circa 10 Minuten bei Raumtemperatur gerührt.
Nach Zugabe von 200 mmol Oxalylchlorid wurde das Reaktionsgemisch für 15 Minuten unter Rückfluß erhitzt und anschließend das überschüssige Oxalylchlorid sowie das Lösungsmittel am Rotationsverdampfer entfernt.
Der so erhaltene Rückstand wurde in 200 ml Diethylether aufgenommen, langsam mit 100 mmol des entsprechenden Amins versetzt und das so erhaltene Reaktionsgemisch zwei Stunden unter Rückfluß erhitzt. Nach dem Abkühlen wurde das Reaktionsgemisch dreimal mit jeweils 50 ml Wasser gewaschen. Die organische Phase wurde abgetrennt, über Magnesiumsulfat getrocknet und am Rotationsverdampfer eingeengt. Das so erhaltene Rohprodukt wurde ohne weitere Aufreinigung in den nachfolgenden Syntheseschritten eingesetzt.

Die nach der vorstehenden allgemeinen Synthesevorschrift hergestellten Verbindungen der allgemeinen Formel II sind in der nachfolgenden Tabelle 1 wiedergegeben:

**Tabelle 1:**

| Verbindung | **R⁴** | **R⁵** | **R⁶** |
|---|---|---|---|
| II-1 | -(CH₂)₂-O-(CH₂)₂- | | CH₃ |
| II-2 | N-Methylpiperazinyl | | CH₃ |
| II-3 | N-Phenylpiperazinyl | | CH₃ |
| II-4 | -(CH₂)₅- | | CH₃ |
| II-5 | -(CH₂)₄- | | CH₃ |
| II-6 | (CH₂)₂Phenyl | H | CH₃ |
| II-7 | CH₂Phenyl | CH₃ | CH₃ |
| II-8 | CH₂Phenyl | CH(CH₃)₂ | CH₃ |
| II-9 | CH₂Phenyl | CH₂Phenyl | CH₃ |
| II-10 | CH₂-2-Pyridin | H | CH₃ |
| II-11 | CH₂-3-Pyridin | H | CH₃ |
| II-12 | CH₂-4-Fluorphenyl | H | CH₃ |
| II-13 | CH₂-2-Furyl | H | CH₃ |
| II-14 | CH₂-2-Methoxy-phenyl | H | CH₃ |
| II-15 | (CH₂)₂-4-Methoxyphenyl | H | CH₃ |
| II-16 | (CH₂)₂-3,4-Dimethoxyphenyl | H | CH₃ |
| II-17 | Cyclopropyl | H | CH₃ |
| II-18 | Cyclohexyl | H | CH₃ |
| II-19 | Cyclohexyl | CH₃ | CH₃ |
| II-20 | Cyclohexyl | Cyclohexyl | CH₃ |
| II-21 | Cyclooctyl | H | CH₃ |
| II-22 | (CH₂)₃CH₃ | H | CH₃ |
| II-23 | (CH₂)₃CH₃ | CH₃ | CH₃ |
| II-24 | (CH₂)₂-morpholino | H | CH₃ |
| II-25 | (CH₂)₃-morpholino | H | CH₃ |

Die Struktur der Verbindungen II-1 bis II-25 wurde jeweils mit Hilfe der ¹H-NMR-Spektroskopie bestimmt. Im folgenden sind die chemischen Verschiebungen ausgewählter Verbindungen wiedergegeben.

### II-1)

### (1-Methyl-1H-pyrrol-2-yl)-morpholin-4-yl-methanon

δ (DMSO, 300 MHz) = 3,58 - 3,66 (m, 8 H, N(C*H*₂CH₂)₂O, N(CH₂C*H*₂)₂O); 3,69 (s, 3 H, NC*H*₃); 5,98 - 6,05 (m, 1 H, N(CH₃)]-CHC*H*C*H*C-[); 6,24 - 6,32 (m, 1 H, N(CH₃)-]-CHC*H*C*H*C-[); 6.78 - 6,88 (m, 1 H, N(CH₃)]-C*H*CHCHC-[).

### II-2)

### (4-Methyl-piperazin-1-yl)-(1-methyl-1H-pyrrol-2-yl)-methanon

δ (DMSO, 300 MHz) = 2,21 (s, 3 H, N-CH₃); 2,28 - 2,39 (m, 4 H, N(C*H*₂CH₂)₂NMe); 3,57 - 3,64 (m, 4 H, N(CH₂C*H*₂)₂NMe); 3,66 (s, 3 H, NC*H*₃); 6,01 (dd, 1 H, *J* = 3,7 Hz, *J* = 1,5 Hz, N(CH₃)]-CHC*H*C*H*C-[); 6,26 (dd, 1 H, *J* = 1,5 Hz, *J* = 4,5 Hz, N(CH₃)]-CHC*H*C*H*C-[); 6,82 - 6,85 (m, 1 H, N(CH₃)-]-CHC*H*C*H*C-[).

### II-3)

### (1-Methyl-1H-pyrrol-2-yl)-(4-phenyl-piperazin-1-yl)-methanon

δ (DMSO, 300 MHz) = 3,13 - 3,23 (m, 4 H, N(C*H*₂CH₂)₂NPh); 3,70 (s, 3 H, NC*H*₃); 3,73 - 3,83 (m, 4 H, N(CH₂C*H*₂)₂NPh); ); 6,04 (dd, 1 H, *J* = 2,6 Hz, *J* = 3,7 Hz, N(CH₃)]-CHC*H*C*H*C-[); 6,34 (dd, 1 H, *J* =1,5 Hz, *J* = 3,7 Hz, N(CH₃)]-CHC*H*C*H*C-[); 6,77 - 6,89 (m, 2 H, N(CH₃)-]-CHC*H*C*H*C-[, Ph); 6,91 - 6,99 (m, 2 H, Ph); 7,17 - 7,28 (m, 2 H, Ph).

### II-4)

### (1-Methyl-1H-pyrrol-2-yl)-piperidin-1-yl-methanon

δ (DMSO, 300 MHz) = 1,45 -1,68 (m, 6 H, N-[(C*H*₂)₂-C*H*₂-(C*H*₂)₂-[); 3,51 - 3,62 (m, 4 H, N-[(C*H*₂)₂-CH₂-(C*H*₂)₂-[); 3,65 (s, 3 H, NC*H*₃); 5,97 - 6,03 (m, 1 H, N(CH₃)-]-CHC*H*C*H*C-[); 6,21 - 6,25 (m, 1 H, N(CH₃)]-CHC*H*C*H*C-[); 6,79 - 6,84 (m, 1 H, N(CH₃)]-C*H*CHCHC-[).

### II-6)

### 1-Methyl-1H-pyrrol-2-carbonsäurephenethyl-amid

δ (DMSO, 300 MHz) = 2,73 - 2,88 (m, 2 H, CH₂C*H*₂Ph); 3,34 - 3,48 (m, 2 H, C*H*₂CH₂Ph); 3,84 (s, 3 H, NC*H*₃); 5,92 - 6,01 (m, 1 H, N(CH₃)]-CHC*H*C*H*C-[); 6,65 - 6,75 (m, 1 H, N(CH₃)-]-CHC*H*C*H*C-[); 6,75 - 6,84 (m, 1 H, N(CH₃)-]-C*H*CHCHC-[); 7,10 - 7,36 (m, 5 H, Ph); 7,95 - 8,08 (m, 1 H, NH).

### II-7)

### 1-Methyl-1H-pyrrol-2-carbonsäure-benzyl-methyl-amid

δ (DMSO, 300 MHz) = 2,99 (br. s, 3 H, NC*H*₃Bn); 3,71 (s, 3 H, NC*H*₃); 4,65 - 4,75 (m, 2 H, C*H*₂Ph); 5,96 - 6,04 (m, 1 H, N(CH₃)]-CHC*H*C*H*C-[); 6,28 - 6,41 (m, 1 H, N(CH₃)-]-CHC*H*C*H*C-[); 6,87 - 6,93 (m, 1 H, N(CH₃)-]-CHC*H*C*H*C-[); 7,18 - 7,43 (m, 5 H, Ph).

### II-8)

### 1-Methyl-1H-pyrrol-2-carbonsäure-benzyl-isopropyl-amid

δ (DMSO, 300 MHz) = 1,15 (d, 3 H, *J* = 6,8 Hz, CH(C*H*₃)₂); 3,67 (s, 3 H, NC*H*₃); 4,52 - 4,67 (m, 3 H, C*H*₂Ph, CH(C*H*₃)₂); 5,95 - 6,03 (m, 1 H, N(CH₃)]-CHC*H*C*H*C-[); 6,25 - 6,34 (m, 1 H, N(CH₃)-]-CHC*H*C*H*C-[); 6,80 - 6,86 (m, 1 H, N(CH₃)-]-CHC*H*C*H*C-[); 7,15 - 7,36 (m, 5 H, Ph).

### II-9)

### 1-Methyl-1H-pyrrol-2-carbonsäure-dibenzylamid

δ (DMSO, 300 MHz) = 3,73 (s, 3 H, NC*H*₃); 4,58 - 4,72 (m, 4 H, C*H*₂Ph); 5,95 (dd, 1 H, *J* = 2,6 Hz, *J* = 6,0 Hz, N(CH₃)]-CHC*H*C*H*C-[); 6,23 - 6,29 (m, 1 H, N(CH₃)-]-CHC*H*C*H*C-[); 6,88 - 6,93 (m, 1 H, N(CH₃)-]-CHC*H*C*H*C*-*[); 7,16 - 7,42 (m, 10 H, Ph).

### II-10)

### 1-Methyl-1H-pyrrol-2-carbonsäure-(pyridin-2-ylmethyl)-amid

δ (DMSO, 300 MHz) = 3,85 (s, 3 H, NC*H*₃); 4,49 (d, 2 H, *J* = 6,0 Hz, C*H*₂Pyridin); 5,99 - 6,04 (m, 1 H, N(CH₃)]-CHC*H*C*H*C-[); 6,83 - 6,89 (m, 2 H, N(CH₃)-]-CHC*H*C*H*C-[); 7,18 - 7,25 (m, 1 H, Pyridin); 7,30 (d, 1 H, *J* = 7,9 Hz, Pyridin); 7,72 (dt, 1 H, *J* = 1,9 Hz, *J* = 7,5 Hz, Pyridin); 8,46 - 8,52 (m, 1 H, Pyridin); 8,52 - 8,59 (m, 1 H, NH).

### II-11)

### 1-Methyl-1H-pyrrol-2-carbonsäure-(pyridin-3-ylmethyl)-amid

δ (DMSO, 300 MHz) = 3,85 (s, 3 H, NC*H*₃); 4,42 (d, 2 H, *J* = 6,0 Hz, C*H*₂Pyridin); 5,98 - 6,02 (m, 1 H, N(CH₃)]-CHC*H*C*H*C-[); 6,80 - 6,85 (m, 1 H, N(CH₃)-]-C*H*C*H*C*H*C-[); 6,85 - 6,88 (m, 1 H, N(CH₃)-]-C*H*C*H*C*H*C-[); 7,32 (dd, 1 H, *J* = 4,9 Hz, *J* = 7,5 Hz, Pyridin); 7,65 - 7,71 (m, 1 H, Pyridin); 8,42 - 8,45 (m, 1 H, Pyridin); 8,51 - 8,58 (m, 2 H, Pyridin, NH).

### II-12)

### 1-Methyl-1H-pyrrol-2-carbonsäure-4-fluoro-benzylamid

δ (DMSO, 300 MHz) = 3,84 (s, 3 H, NC*H*₃); 4,37 (d, 2 H, *J* = 6,0 Hz, C*H*₂4-FPh); 5,98 - 6,03 (m, 2 H, N(CH₃)]-CHC*H*C*H*C-[); 6,88 - 6,90 (m, 1 H, N(CH₃)]-CHC*H*C*H*C-[); 7,09 - 7,17 (m, 2 H, 4-FPh); 7,28 - 7,36 (m, 2 H, 4-FPh); 8,47 - 8,58 (m, 1 H, NH).

### II-13)

### 1-Methyl-1H-pyrrol-2-carbonsäure(furan-2-ylmethyl)-amid

δ (DMSO, 300 MHz) = 3,85 (s, 3 H, NC*H*₃); 4,38 (d, 2 H, *J* = 5,7 Hz, CONHC*H*₂); 5,95 - 6,03 (m, 1 H, O]-CHC*H*C*H*C-[); 6,19 - 6,26 (m, 1 H, N(CH₃)]-CHC*H*C*H*C-[); 6,34 - 6,40 (m, 1 H, O]-CHC*H*C*H*C-[); 6,78 - 6,84 (m, 1 H, N(CH₃)]-CHC*H*C*H*C-[); 6,85 - 6,90 (m, 1 H, N(CH₃)]-CHC*H*C*H*C-[); 7,49 - 7,56 (m, 1 H, O]-CHC*H*C*H*C-[); 8,36 - 8,46 (m, 1 H, NH).

### II-14)

### 1-Methyl-1H-pyrrol-2-carbonsäure-2-methoxy-benzylamid

δ (DMSO, 300 MHz) = 3,83 (s, 3 H, NC*H*₃); 3,85 (s, 3 H, OC*H*₃); 4,38 (d, 2 H, *J* = 6,0 Hz, C*H*₂-*o*-OMePh); 5,96 - 6,03 (m, 1 H, N(CH₃)]-CHC*H*C*H*C-[); 6,81 - 7,00 (m, 4 H, N(CH₃)-]-CHC*H*C*H*C-[, *p*-OMePh); 7,13 - 7,27 (m, 2 H, p-OMePh); 8,22 - 8,32 (m, 1 H, NH).

### II-15)

### 1-Methyl-1H-pyrrol-2-carbonsäure-[2-(4-methoxy-phenyl)-ethyl]-amid

δ (DMSO, 300 MHz) = 2,72 (d, 1 H, *J* = 7,1 Hz, CH₂C*H*₂-*p*-OMePh); 2,75 (d, 1 H, *J* = 7,9 Hz, CH₂C*H*₂-*p*-OMePh); 3,27 - 3,41 (m, 2 H, CH₂C*H*₂-*p*-OMePh); 3,72 (s, 3 H, NC*H*₃); 3,83 (s, 3 H, OC*H*₃); 5,93 - 6,01 (m, 1 H, N(CH₃)]-CHC*H*C*H*C-[); 6,66 - 6,73 (m, 1 H, N(CH₃)-]-CHC*H*C*H*C-[); 6,78 - 6,88 (m, 3 H, N(CH₃)-]-C*H*CHCHC-[, p-OMePh); 7,13 (d, 2 H, *J* = 8,7 Hz, -[, p-OMePh); 7,95 - 8,02 (m, 1 H, NH).

### II-16)

### 1-Methyl-1H-pyrrol-2-carbonsäure-[2-(3,4-dimethoxy-phenyl)-ethyl]-amid

δ (DMSO, 300 MHz) = 2,73 (d, 1 H, *J* = 7,5 Hz, CH₂C*H*₂(3,4-(OMe)₂Ph); 2,75 (d, 1 H, *J* = 7,5 Hz, CH₂C*H*₂(3,4-(OMe)₂Ph); 3,34 - 3,45 (m, 2 H, C*H*₂CH₂(3,4-(OMe)₂Ph); 3,73 (s, 3 H, NC*H*₃); 5,97 (dd, 1 H, *J* = 2,6 Hz, *J* = 3,0 Hz N(CH₃)]-CHC*H*C*H*C-[); 6,70 - 6,75 (m, 2 H, N(CH₃)]-CHC*H*C*H*C-[); 6,80 - 6,85 (m, 3 H, (3,4-(OMe)₂Ph); 7,91 - 8,01 (m, 1 H, NH).

### II-17)

### 1-Methyl-1H-pyrrol-2-carbonsäurecyclopropylamid

δ (DMSO, 300 MHz) = 0,47 - 0,68 (m, 4 H, ]-C*H*₂CHC*H*₂-[); 2,68 - 2,78 (m, 1 H, ]-CH₂C*H*CH₂-[); 3,84 (s, 3 H, NC*H*₃); 5,91 - 5,99 (m, 1 H, N(CH₃)-]-CHC*H*C*H*C-[); 6,68 - 6,75 (m, 1 H, N(CH₃)]-CHC*H*C*H*C-[); 6,77 - 6,84 (m, 1 H, N(CH₃)]-C*H*CHCHC-[); 7,89 (br. s, 1 H, NH).

### II-18)

### 1-Methyl-1H-pyrrol-2-carbonsäurecyclohexylamid

1,02 -1,39 (m, 6 H, NH]-CH(CH₂)₂(C*H*₂)₂C*H*₂-[); 1,54 - 1,88 (m, 4 H, NH]-CH(C*H*₂)₂(CH₂)₂CH₂-[); 3,60 - 3,78 (m, 1 H, NH]-C*H*(CH₂)₂(CH₂)₂CH₂-[); 3,83 (s, 3 H, NC*H*₃); 5,92 - 6,00 (m, 1 H, N(CH₃)]-CHC*H*C*H*C-[); 6,69 - 6,87 (m, 2 H, N(CH₃)]-CHC*H*C*H*C-[); 7,51 - 7,66 (m, 1 H, NH).

### II-19)

### 1-Methyl-1H-pyrrol-2-carbonsäure-cyclohexyl-methyl-amid

δ (DMSO, 300 MHz) = 0,88 -1,36 (m, 4 H, N]-CH(CH₂)₂(C*H*₂)₂C*H*₂-[); 1,44 -1,85 (m, 6 H, N]-CH(C*H*₂)₂(CH₂)₂CH₂-[); 2,88 (s, 3 H, N-CH₃); 3,64 (s, 3 H, NC*H*₃); 4,03 - 4,19 (m, 1 H, N]-C*H*(CH₂)₂(CH₂)₂CH₂-[); 5,97-6,04 (m, 1 H, N(CH₃)]-CHC*H*C*H*C-[); 6,19 - 6,29 (m, 1 H, N(CH₃)]-CHC*H*C*H*C-[); 6,79 - 6,85 (m, 1 H, N(CH₃)]-CHC*H*C*H*C-[).

### II-20)

### 1-Methyl-1H-pyrrol-2-carbonsäure-dicyclohexylamid

δ (DMSO, 300 MHz) = 1,03 -1,33 (m, 6 H, N]-CH(CH₂)₂(C*H*₂)₂C*H*₂-[); 1,49 -1,83 (m, 11 H, N]-CH(C*H*₂)₂(CH₂)₂CH₂-[); 1,84-2,14 (m, 3 H, N]-CH(C*H*₂)₂(CH₂)₂CH₂-[); 3,38 - 3,55 (m, 2 H, N]-C*H*(CH₂)₂(CH₂)₂CH₂-[); 3,64 (s, 3 H, NC*H*₃); 5,93 - 6,00 (m, 1 H, N(CH₃)]-CHC*H*C*H*C-[); 6,03-6,11 (m, 1 H, N(CH₃)]-CHC*H*C*H*C-[); 6,73-6,80 (m, 1 H, N(CH₃)]-CHC*H*C*H*C-[).

### II-21)

### 1-Methyl-1H-pyrrol-2-carbonsäurecyclooctylamid

δ (DMSO, 300 MHz) = 1,38 -1,77 (m, 16 H, NCH(C*H*₂)₇); 3,82 (s, 3 H, NC*H*₃); 3,88-4,02 (m, 1 H, NC*H*(CH₂)₇); 5,93 - 5,99 (m, 1 H, N(CH₃)]-CHC*H*C*H*C-[); 6,75-6,83 (m, 2 H, N(CH₃)-]-CHC*H*C*H*C-[); 7,68 - 7,59 (m, 1 H, NH).

### II-22)

### 1-Methyl-1H-pyrrol-2-carbonsäurebutylamid

δ (DMSO, 300 MHz) = 0,90 (t, 3 H, *J* = 7,2 Hz, C*H*₃CH₂CH₂CH₂); 1,21 - 1,40 (m, 2 H, CH₃C*H*₂CH₂CH₂); 1,40 -1,56 (m, 2 H, CH₃CH₂C*H*₂CH₂); 3,17 (dd, 2 H, *J* = 6,4 Hz, *J* = 13,5 Hz, CH₃CH₂CH₂C*H*₂); 3,83 (s, 3 H, NC*H*₃); 5,93 (m, 1 H, N(CH₃)-]-CHC*H*C*H*C-[); 6,68 - 6,75 (m, 1 H, N(CH₃)]-CHC*H*C*H*C-[); 6,76 - 6,83 (m, 1 H, N(CH₃)]-CHCHCHC-[); 7,78 - 7,93 (m, 1 H, NH).

### II-23)

### 1-Methyl-1H-pyrrol-2-carbonsäure-butyl-methyl-amid

δ (DMSO, 300 MHz) = 0,88 (t, 3 H, *J* = 7,2 Hz, C*H*₃CH₂CH₂CH₂); 1,18 -1,34 (m, 2 H, CH₃C*H*₂CH₂CH₂); 1,47-1,61 (m, 2 H, CH₃CH₂C*H*₂CH₂); 3,01 (br.s, 3 H, NC*H*₃); 3,44 (t, 2 H, *J* = 7,2 Hz, CH₃CH₂CH₂C*H*₂); 3,65 (s, 3 H, NC*H*₃); 5,97 - 6,03 (m, 1 H, N(CH₃)-]-CHC*H*C*H*C-[); 6,25 - 6,32 (m, 1 H, N(CH₃)]-CHC*H*C*H*C-[); 6,81 - 6,83 (m, 1 H, N(CH₃)]-C*H*CHCHC-[).

### II-24)

### 1-Methyl-1H-pyrrol-2-carbonsäure-(3-morpholin-4-yl-ethyl)-amid

δ (DMSO, 300 MHz) = 2,38 - 2,45 (m, 6 H, CONHC*H*₂CH₂, N(C*H*₂CH₂)₂O); 3,27 - 3,34 (m, 2 H, CONHCH₂C*H*₂); 3,54 - 3,62 (m, 4 H, N(CH₂C*H*₂)₂O); 3,83 (s, 3 H, NC*H*₃); 5,98 (dd, 1 H, *J* = 2,6 Hz, *J* = 3,7 Hz, N(CH₃)]-CHC*H*C*H*C-[); 6,69 - 6,71 (m, 1 H, N(CH₃)-]-CHC*H*C*H*C-[); 6,80 - 6,84 (m, 1 H, N(CH₃)-]-CHC*H*C*H*C-[); 7,77 - 7,84 (m, 1 H, NH).

### II-25)

### 1-Methyl-1H-pyrrol-2-carbonsäure-(3-morpholin-4-yl-propyl)-amid

δ (DMSO, 300 MHz) = 1,58 - 1,68 (m, 2 H, CONHCH₂C*H*₂CH₂); 2,23 - 2,42 (m, 6 H, CONHC*H*₂CH₂CH₂, N(C*H*₂CH₂)₂O); 3,17 (d, 1 H, *J* = 6,8 Hz, CONHCH₂CH₂C*H*₂); 3,21 (d, 1 H, *J* = 6,8 Hz, CONHCH₂CH₂C*H*₂); 3,35 - 3,60 (m, 4 H, N(CH₂C*H*₂)₂O); 3,81 (s, 3 H, NC*H*₃); 5,96 - 6,03 (m, 1 H, N(CH₃)]-CHC*H*C*H*C-[); 6,67 - 6,76 (m, 1 H, N(CH₃)-]-CHC*H*C*H*C-[); 6,83 - 6,91 (m, 1 H, N(CH₃)-]-CHC*H*C*H*C-[); 7,90 - 8,03 (m, 1 H, NH).

### B)

### Allgemeine Synthesevorschrift zur Herstellung von Verbindungen der allgemeinen Formel III:

Eine Lösung von einem Äquivalent Acetylchlorid in Diethylether wurde langsam unter Rühren zu der eisgekühlten Lösung bzw. Suspension von einem Äquivalent der jeweiligen Verbindung der allgemeinen Formel V getropft. Anschließend wurde das Reaktionsgemisch für eine Stunde bei Raumtemperatur nachgerührt. Es entstand ein Niederschlag, der unter Stickstoff abgesaugt und anschließend im Ölpumpenvakuum getrocknet wurde. Die so erhaltenen Imminiumsalze der allgemeinen Formel III wurden dann ohne jede weitere Aufreinigung im nachfolgenden Syntheseschritt eingesetzt.

Abweichend von dieser Synthesevorschrift wurden die Verbindungen III-4 und III-5 nicht isoliert, sondern unmittelbar im nachfolgenden Syntheseschritt eingesetzt.

Die nach der vorstehenden allgemeinen Synthesevorschrift hergestellten Verbindungen der allgemeinen Formel III sind in der nachfolgenden Tabelle 2 wiedergegeben:

**Tabelle 2:**

| **Verbindung** | **R¹** | **R²** | **R³** |
|---|---|---|---|
| III-1 | CH₃ | CH₃ | 4-Fluor-phenyl |
| III-2 | CH₃ | CH₃ | 4-Brom-phenyl |
| III-3 | CH₃ | CH₃ | Phenyl |
| III-4 | CH₃ | CH₃ | COOC₂H₅ |
| III-5 | -(CH₂)₅- | | COOC₂H₅ |

### C)

### Allgemeine Synthesevorschrift zur Herstellung der erfindungsgemäßen Verbindungen gemäß Beispiel 1-15:

Die jeweiligen Verbindungen der allgemeinen Formeln II und III wurden in Acetonitril gelöst bzw. die Verbindungen III-4 und III-5 unmittelbar eingesetzt wie gemäß B) erhalten und über Nacht bei Raumtemperatur (ca. 20-25 °C) gerührt. Die so erhaltene Lösung wurde zunächst mit wäßriger Salzsäure angesäuert und nichtbasische Verunreinigungen mit Diethylether extrahiert. Anschließend wurde die wäßrige Phase mit Na₂CO₃-Lösung versetzt und die jeweilige Beispielverbindung mit Diethylether extrahiert. Die so erhaltene etherische Lösung wurde über Magnesiumsulfat getrocknet. Anschließend wurde die jeweilige erfindungsgemäße Verbindung gemäß Beispiel 1-15 mit Hilfe einer ethanolischen ChlorwasserstoffLösung in das entsprechende Hydrochlorid übergeführt und durch Filtration isoliert. Das so erhalte Salz wurde anschließend durch Waschen mit Ethanol gereinigt.

Die jeweils zur Herstellung der erfindungsgemäßen Beispielverbindungen eingesetzten Verbindungen der allgemeinen Formeln II und III sind in der nachfolgenden Tabelle 3 wiedergegeben. **Tabelle 3:**

| **Beispiel** | **Eingesetzte Verbindung der allgemeinen Formel II** | **Eingesetzte Verbindung der allgemeinen Formel III** |
|---|---|---|
| 1 | II-18 | III-1 |
| 2 | II-22 | III-1 |
| 3 | II-5 | III-1 |
| 4 | II-18 | III-2 |
| 5 | II-22 | III-2 |
| 6 | II-5 | III-2 |
| 7 | II-6 | III-2 |
| 8 | II-1 | III-2 |
| 9 | II-18 | III-3 |
| 10 | II-22 | III-3 |
| 11 | II-5 | III-3 |
| 12 | II-22 | III-4 |
| 13 | II-18 | III-4 |
| 14 | II-22 | III-5 |
| 15 | II-18 | III-5 |

Die Struktur der erfindungsgemäßen Beispielverbindungen wurde jeweils mit Hilfe der ¹H-NMR-Spektroskopie bestimmt. Die jeweils erhaltenen Werte sind im folgenden wiedergegeben.

### Beispiel 1:

### 5-[Dimethylamino-(4-fluoro-phenyl)-methyl]-1-methyl-1H-pyrrol-2-carbonsäurecyclohexylamid Hydrochlorid

Das ¹H-NMR zeigt zwei Rotamere im Verhältnis ca. 3 : 1. Es sind nur die Signale der Überschußverbindung angegeben.

δ (DMSO, 600 MHz) = 1,05 - 1,15 (m, 1 H, NH]-CH(CH₂)₂(CH₂)₂C*H*₂-[); 1,18 - 1,32 (m, 5 H, NH]-CH(CH₂)₂(C*H*₂)₂C*H*₂-[); 1,65 -1,85 (m, 4 H, NH]-CH(C*H*₂)₂(CH₂)₂CH₂); 2,61 (d, 3 H, *J* = 4,5 Hz, N(CH₃)₂); 2,79 (d, 3 H, *J* = 4,5 Hz, N(CH₃)₂); 3,60 - 3,72 (m, 1 H, NH]-C*H*(CH₂)₂(CH_{Z})₂CH₂-[); 3,84 (s, 3 H, NC*H*₃); 5,82 (d, 1 H, *J* = 9,1 Hz, C*H*N(CH₃)₂); 6,81 (d, 1 H, *J* = 3,8 Hz, N(CH₃)]-CC*H*C*H*C-[); 6,86 (d, 1 H, *J* = 3,8 Hz, N(CH₃)]-CC*H*C*H*C-[); 7,24 - 7,34 (m, 2 H, *p*-FPh); 7,72 - 7,82 (m, 3 H, *p*-FPh, NH); 11,64 (s, 1 H, HCl).

### Beispiel 2:

### 5-[Dimethylamino-(4-fluoro-phenyl)-methyl]-1-methyl-1H-pyrrol-2-carbonsäurebutylamid Hydrochlorid

Das ¹H-NMR zeigt zwei Rotamere im Verhältnis ca. 3 : 1. Es sind nur die Signale der Überschußverbindung angegeben.

δ (DMSO, 600 MHz) = 0,88 (t, 3 H, *J* = 7,5 Hz, C*H*₃CH₂CH₂CH₂); 1,25 -1,34 (m, 2 H, CH₃C*H*₂CH₂CH₂); 1,40 - 1,48 (m, 2 H, CH₃CH₂C*H*₂CH₂); 2,61 (d, 3 H, *J* = 4,5 Hz, N(CH₃)₂); 2,79 (d, 3 H, *J* = 4,5 Hz, N(CH₃)₂); 3,10 - 3,20 (m, 2 H, CH₃CH₂CH₂C*H*₂); 3,86 (s, 3 H, NC*H*₃); 5,85 (d, 1 H, *J* = 9,1 Hz, C*H*N(CH₃)₂); 6,79 (d, 1 H, *J* = 3,8 Hz, N(CH₃)]-CC*H*C*H*C-[); 6,91 (d, 1 H, *J* = 3,8 Hz, N(CH₃)]-CC*H*C*H*C-[); 7,24 - 7,32 (m, 2 H, *p*-FPh); 7,75 - 7,83 (m, 2 H, *p*-FPh); 8,03 - 8,07 (m, 1 H, NH); 11,76 (s, 1 H, HCl).

### Beispiel 3:

### {5-[Dimethylamino-(4-fluoro-phenyl)-methy)]-1-methyl-1H-pyrrol-2-yl}-pyrrolidin-1-yl-methanon Hydrochlorid

Das ¹H-NMR zeigt zwei Rotamere im Verhältnis ca. 4 : 1. Es sind nur die Signale der Überschußverbindung angegeben.

δ (DMSO, 600 MHz) = 1,72 -1,91 (m, 4 H, N(CH₂)₂(C*H*₂)₂); 2,61 (d, 3 H, *J* = 4,5 Hz, N(CH₃)₂); 2,79 (d, 3 H, *J* = 4,5 Hz, N(CH₃)₂); 3,35 - 3,59 (m, 4 H, N(C*H*₂)₂(CH₂)₂); 3,71 (s, 3 H, NC*H*₃); 5,87 (d, 1 H, *J* = 9,1 Hz, C*H*N(CH₃)₂); 6,57 (d, 1 H, *J* = 3,8 Hz, N(CH₃)]-CC*H*C*H*C-[); 6,95 (d, 1 H, *J* = 3,8 Hz, N(CH₃)]-CC*H*C*H*C-[); 7,27 - 7,33 (m, 2 H, *p*-FPh); 7,78 - 7,88 (m, 2 H, *p*-FPh); 11,85 (s, 1 H, HCl).

### Beispiel 4:

### 5-[Dimethylamino-(4-bromo-phenyl)-methyl]-1-methyl-1H-pyrrol-2-carbonsäurecyclohexylamid Hydrochlorid

Das ¹H-NMR zeigt zwei Rotamere im Verhältnis ca. 4,5 : 1. Es sind nur die Signale der Überschußverbindung angegeben.

δ (DMSO, 600 MHz) = 1,08 - 1,16 (m, 1 H, NH]-CH(CH₂)₂(CH₂)₂C*H*₂-[); 1,19 - 1,34 (m, 5 H, NH]-CH(CH₂)₂(C*H*₂)₂C*H*₂-[); 1,53-1,64 (m, 1 H, NH]-CH(C*H*₂)₂(CH₂)₂CH₂); 1,65 - 1,82 (m, 3 H, NH]-CH(C*H*₂)₂(CH₂)₂CH₂); 2,63 (d, 3 H, *J* = 4,5 Hz, N(CH₃)₂); 2,78 (d, 3 H, *J* = 4,5 Hz, N(CH₃)₂); 3,64 - 3,72 (m, 1 H, NH]-C*H*(CH₂)₂(CH₂)₂CH₂-[); 3,85 (s, 3 H, NC*H*₃); 5,84 (d, 1 H, *J* = 9,1 Hz, C*H*N(CH₃)₂); 6,81 (d, 1 H, *J* = 4,5 Hz, N(CH₃)]-CC*H*C*H*C-[); 6,87 (d, 1 H, *J* = 4,5 Hz, N(CH₃)]-CC*H*C*H*C-[); 7,63 - 7,67 (m, 2 H, *p*-BrPh); 7,67 - 7,72 (m, 2 H, *p*-BrPh); 7,81 (d, 1 H, *J* = 8,3 Hz, NH); 11,81 (s, 1 H, HCl).

### Beispiel 5:

### 5-[Dimethylamino-(4-bromo-phenyl)-methyl]-1-methyl-1H-pyrrol-2-carbonsäurebutylamid Hydrochlorid

Das ¹H-NMR zeigt zwei Rotamere im Verhältnis ca. 3 : 1. Es sind nur die Signale der Überschußverbindung angegeben.

δ (DMSO, 600 MHz) = 0,88 (t, 3 H, *J* = 7,2 Hz, C*H*₃CH₂CH₂CH₂); 1,26 - 1,33 (m, 2 H, CH₃C*H*₂CH₂CH₂); 1,41 -1,48 (m, 2 H, CH₃CH₂C*H*₂CH₂); 2,63 (d, 3 H, *J* = 3,8 Hz, N(CH₃)₂); 2,78 (d, 3 H, *J* = 4,5 Hz, N(CH₃)₂); 3,11 - 3,18 (m, 2 H, CH₃CH₂CH₂C*H*₂); 3,86 (s, 3 H, NC*H*₃); 5,85 (d, 1 H, *J* = 9,1 Hz, C*H*N(CH₃)₂); 6,79 (d, 1 H, *J* = 3,8 Hz, N(CH₃)]-CC*H*C*H*C-[); 6,90 (d, 1 H, *J* = 3,8 Hz, N(CH₃)]-CC*H*C*H*C-[); 7,64 - 7,68 (m, 2 H, *p*-BrPh); 7,68 - 7,73 (m, 2 H, *p*-BrPh); 8,03 - 8,08 (m, 1 H, NH); 11,86 (s, 1 H, HCl).

### Beispiel 6:

### {5-[(4-Bromo-phenyl)-dimethylamino-methyl]-1-methyl-1H-pyrrol-2-y/}-pyrrolidin-1-ylmethanon Hydrochlorid

Das ¹H-NMR zeigt zwei Rotamere im Verhältnis ca. 4 : 1. Es sind nur die Signale der Überschußverbindung angegeben.

δ (DMSO, 600 MHz) = 1,74 -1,92 (m, 4 H, N(CH₂)₂(C*H*₂)₂); 2,63 (d, 3 H, *J* = 4,5 Hz, N(CH₃)₂); 2,78 (d, 3 H, *J* = 4,5 Hz, N(CH₃)₂); 3,34 - 3,59 (m, 4 H, N(C*H*₂)₂(CH₂)₂); 3,71 (s, 3 H, NC*H*₃); 5,82 - 5,89 (m, 1 H, C*H*N(CH₃)₂); 6,56 (d, 1 H, *J* = 3,8 Hz, N(CH₃)]-CC*H*C*H*C-[); 6,92 (d, 1 H, *J* = 3,8 Hz, N(CH₃)]-CC*H*C*H*C-[); 7,66 (d, 2 H, *J* = 8,3 Hz, *p*-BrPh); 7,73 (d, 2 H, *J* = 8,3 Hz, *p*-FPh); 11,86 (s, 1 H, HCl).

### Beispiel 7:

### 5-[(4-Bromo-phenyl)-dimethylamino-methyl]-1-methyl-1H-pyrrol-2-carbonsäurephenethyl-amid Hydrochlorid

Das ¹H-NMR zeigt zwei Rotamere im Verhältnis ca. 3 : 1. Es sind nur die Signale der Überschußverbindung angegeben.

δ (DMSO, 600 MHz) = 2,59 - 2,61 (m, 2 H, CH₂C*H*₂Ph); 2,67 - 2,71 (m, 2 H, C*H*₂CH₂Ph); 2,75 - 2,79 (m, 6 H, N(CH₃)₂); 3,85 (s, 3 H, NC*H*₃); 5,82 (d, 1 H, *J* = 9,1 Hz, C*H*N(CH₃)₂); 6,77 (d, 1 H, *J* = 3,8 Hz, N(CH₃)]-CC*H*C*H*C-[); 6,86 (d, 1 H, *J* = 3,8 Hz, N(CH₃)]-CC*H*C*H*C-[); 7,17 - 7,31 (m, 7 H, *p*-BrPh, Ph); 7,63 - 7,71 (m, 2 H, *p-*BrPh); 8,19 - 8,23 (m, 1 H, NH); 11,66 (s, 1 H, HCl).

### Beispiel 8:

### {5-[(4-Bromo-phenyl)-dimethylamino-methyl]-1-methyl-1H-pyrrol-2-yl}-morpholin-4-yl-methanon Hydrochlorid

δ (DMSO, 600 MHz) = 2,59 - 2,69 (m, 4 H, N(C*H*₂C*H*₂)₂O); 2,74 - 2,83 (m, 4 H, N(CH₂C*H*₂)₂O); 3,51 - 3,59 (m, 6 H, N(CH₃)₂); 3,63 (s, 3 H, NC*H*₃); 5,78 - 5,85 (m, 1 H, C*H*N(CH₃)₂); 6,39 (d, 1 H, *J* = 3,8 Hz, N(CH₃)]-CC*H*C*H*C-[); 6,82 (d, 1 H, *J* = 3,8 Hz, N(CH₃)]-CC*H*C*H*C-[); 7,65 - 7,71 (m, 4 H, *p*-BrPh); 11,44 (s, 1 H, HCl).

### Beispiel 9:

### 5-(Dimethylamino-phenyl-methyl)-1-methyl-1H-pyrrol-2-carbonsäure-cyclohexylamid Hydrochlorid

Das ¹H-NMR zeigt zwei Rotamere im Verhältnis ca. 4 : 1. Es sind nur die Signale der Überschußverbindung angegeben.

δ (DMSO, 600 MHz) = 1,08 - 1,15 (m, 1 H, NH]-CH(CH₂)₂(CH₂)₂C*H*₂-[); 1,21 - 1,33 (m, 5 H, NH]-CH(CH₂)₂(CH₂)₂C*H*₂-[); 1,65 -1,85 (m, 4 H, NH]-CH(C*H*₂)₂(CH₂)₂CH₂); 2,61 (d, 3 H, *J* = 4,5 Hz, N(CH₃)₂); 2,78 (d, 3 H, *J* = 4,5 Hz, N(CH₃)₂); 3,61 - 3,70 (m, 1 H, NH]-C*H*(CH₂)₂(CH₂)₂CH₂-[); 3,86 (s, 3 H, NC*H*₃); 5,80 (d, 1 H, *J* = 9,1 Hz, C*H*N(CH₃)₂); 6,81 (d, 1 H, *J* = 4,5 Hz, N(CH₃)]-CC*H*C*H*C-[); 6,89 (d, 1 H, *J* = 3,8 Hz, N(CH₃)]-CC*H*C*H*C-[); 7,36 - 7,41 (m, 1 H, Ph); 7,42 - 7,49 (m, 2 H, Ph); 7,42 - 7,49 (m, 2 H, Ph); 7,80 (d, 1 H, *J* = 8,3 Hz, NH);11,74 (s, 1 H, HCl).

### Beispiel 10:

### 5-(Dimethylamino-phenyl-methyl)-1-methyl-1H-pyrrol-2-carbonsäure-butylamid Hydrochlorid

Das ¹H-NMR zeigt zwei Rotamere im Verhältnis ca. 4 : 1. Es sind nur die Signale der Überschußverbindung angegeben.

δ (DMSO, 600 MHz) = 0,88 (t, 3 H, *J* = 7,6 Hz, C*H*₃CH₂CH₂CH₂); 1,26 - 1,33 (m, 2 H, CH₃C*H*₂CH₂CH₂); 1,40 - 1,48 (m, 2 H, CH₃CH₂C*H*₂CH₂); 2,61 (d, 3 H, *J* = 4,5 Hz, N(CH₃)₂); 2,78 (d, 3 H, *J* = 4,5 Hz, N(CH₃)₂); 3,10 - 3,20 (m, 2 H, CH₃CH₂CH₂C*H*₂); 3,87 (s, 3 H, NC*H*₃); 5,80 (d, 1 H, *J* = 9,1 Hz, C*H*N(CH₃)₂); 6,79 (d, 1H, *J* = 3,8 Hz, N(CH₃)]-CC*H*CHC-[); 6,90 (d, 1 H, *J* = 3,8 Hz, N(CH₃)]-CC*H*C*H*C-[); 7,36 - 7,42 (m, 1 H, Ph); 7,42 - 7,48 (m, 2 H, Ph); 7,69 - 7,78 (m, 2 H, Ph); 8,01 - 8,07 (m, 1 H, NH); 11,74 (s, 1 H, HCl).

### Beispiel 11:

### [5-(Dimethylamino-phenyl-methyl)-1-methyl-1H-pyrrol-2-yl]-pyrrolidin-1-yl-methanon Hydrochlorid

Das ¹H-NMR zeigt zwei Rotamere im Verhältnis ca. 4 : 1. Es sind nur die Signale der Überschußverbindung angegeben.

δ (DMSO, 600 MHz) = 1,73 - 1,92 (m, 4 H, N(CH₂)₂(C*H*₂)₂); 2,61 (d, 3 H, *J* = 4,5 Hz, N(CH₃)₂); 2,79 (d, 3 H, *J* = 4,5 Hz, N(CH₃)₂); 3,34 - 3,61 (m, 4 H, N(C*H*₂)₂(CH₂)₂); 3,73 (s, 3 H, NC*H*₃); 5,81 (d, 1 H, *J* = 9,1 Hz, C*H*N(CH₃)₂); 6,56 (d, 1 H, *J* = 3,8 Hz, N(CH₃)]-CC*H*C*H*C-[); 6,93 (d, 1 H, *J* = 3,8 Hz, N(CH₃)]-CC*H*C*H*C); 7,38 - 7,52 (m, 3 H, Ph); 7,72 - 7,81 (m, 2 H, Ph); 11,75 (s, 1 H, HCl).

### Beispiel 12:

### (5-Butylcarbamoyl-1-methyl-1H-pyrrol-2-yl)-dimethylamino-essigsäureethylester Hydrochlorid

δ (DMSO, 600 MHz) = 0,90 (t, 3 H, *J* = 7,5 Hz, C*H*₃CH₂CH₂CH₂); 1,22 (t, 3 H, *J* = 6,8 Hz, C*H*₃CH₂CO₂); 1,28 -1,34 (m, 2 H, CH₃C*H*₂CH₂CH₂); 1,44 - 1,49 (m, 2 H, CH₃CH₂C*H*₂CH₂); 2,62 (br.s, 3 H, N(CH₃)₂); 2,79 (br.s, 3 H, N(CH₃)₂); 3,12 - 3,22 (m, 2 H, CH₃CH₂CH₂C*H*₂); 3,85 (s, 3 H, NC*H*₃); 4,17 - 4,24 (m, 1 H, CH₃C*H*₂CO₂); 4,24 - 4,32 (m, 1 H, CH₃C*H*₂CO₂); 5,22 - 5,32 (m, 1 H, C*H*N(CH₃)₂); 6,79 - 6,84 (m, 1 H, N(CH₃)]-CC*H*C*H*C-[); 7,11 - 7,17 (m, 1 H, N(CH₃)]-CC*H*C*H*C); 8,10 - 8,18 (m, 1 H, NH); 10,59 (s, 1 H, HCl).

### Beispiel 13:

### (5-Cyclohexylcarbamoyl-1-methyl-1H-pyrrol-2-yl)-dimethylaminoessigsäureethylester Hydrochlorid

Das ¹H-NMR zeigt zwei Rotamere im Verhältnis ca. 4 : 1. Es sind nur die Signale der Überschußverbindung angegeben.

δ (DMSO, 600 MHz) = 1,06 - 1,15 (m, 1 H, NH]-CH(CH₂)₂(CH₂)₂C*H*₂-[); 1,18 (t, 3 H, *J* = 6,8 Hz, C*H*₃CH₂CO₂); 1,25 - 1,34 (m, 5 H, NH]-CH(CH₂)₂(C*H*₂)₂C*H*₂-[); 1,67 - 1,83 (m, 4 H, NH]-CH(C*H*₂)₂(CH₂)₂CH₂); 2,60 (br.s, 3 H, N(CH₃)₂); 2,94 (br.s, 3 H, N(CH₃)₂); 3,47 - 3,62 (m, 1 H, NH]-C*H*(CH₂)₂(CH₂)₂CH₂-[); 3,95 (s, 3 H, NC*H*₃); 4,16 - 4,32 (m, 1 H, CH₃C*H*₂CO₂); 5,60 - 5,71 (m, 1 H, C*H*N(CH₃)₂); 6,30 - 6,40 (m, 1 H, N(CH₃)]-CC*H*C*H*C-[); 6,79 - 6,85 (m, 1 H, N(CH₃)]-CC*H*C*H*C-[); 7,89 - 7,99 (m, 1 H, NH); 11,08 (s, 1 H, HCl).

### Beispiel 14:

### (5-Butylcarbamoyl-1-methyl-1H-pyrrol-2-yl)-piperidin-1-yl-essigsäureethylester Hydrochlorid

δ (DMSO, 600 MHz) = 0,90 (t, 3 H, *J* = 7,2 Hz, C*H*₃CH₂CH₂CH₂); 1,22 (t, 3 H, *J* = 7,2 Hz, C*H*₃CH₂CO₂); 1,26 - 1,38 (m, 2 H, CH₃C*H*₂CH₂CH₂); 1,42 - 1,51 (m, 2 H, CH₃CH₂C*H*₂CH₂); 1,63 - 1,71 (m, 2 H, N(CH₂)₂(CH₂)₂C*H*₂); 1,72 - 1,90 (m, 4 H, N(CH₂)₂(C*H*₂)₂C*H*₂); 2,89 - 3,02 (m, 2 H, N(C*H*₂)₂(CH₂)₂CH₂); 3,10 - 3,24 (m, 2 H, N(C*H*₂)₂(CH₂)₂CH₂); 3,37 - 3,41 (m, 1 H, CH₃CH₂CH₂C*H*₂); 3,42 - 3,49 (m, 1 H, CH₃CH₂CH₂C*H*₂); 3,84 (s, 3 H, NC*H*₃); 4,15 - 4,22 (m, 1 H, CH₃C*H*₂CO₂); 4,25 - 4,32 (m, 1 H, CH₃C*H*₂CO₂); 5,19 - 5,25 (m, 1 H, C*H*N(CH₂)₂(CH₂)₂CH₂); 6,77 - 6,83 (m, 1 H, N(CH₃)]-CC*H*C*H*C-[); 7,09 - 7,15 (m, 1 H, N(CH₃)]-CC*H*C*H*C); 8,11 - 8,18 (m, 1 H, NH); 10,38 (s, 1 H, HCl).

### Beispiel 15:

### (5-Cyclohexylcarbamoyl-1-methyl-1H-pyrrol-2-yl)-piperidin-1-yl-essigsäureethylester Hydrochlorid

δ (DMSO, 600 MHz) = 1,05 - 1,16 (m, 1 H, NH]-CH(CH₂)₂(CH₂)₂C*H*₂-[); 1,22 (t, 3 H, *J* = 6,8 Hz, C*H*₃CH₂C₂); 1,24 - 1,36 (m, 5 H, NH]-CH(CH₂)₂(C*H*₂)₂C*H*₂-[); 1,56 -1,86 (m, 10 H, NH]-CH(C*H*₂)₂(CH₂)₂CH₂, N(CH₂)₂(CH₂)₂C*H*₂, N(CH₂)₂(C*H*₂)₂C*H*₂); 2,63 - 2,72 (m, 1 H, N(C*H*₂)₂(CH₂)₂CH₂); 2,91 - 2,98 (m, 1 H, N(C*H*₂)₂(CH₂)₂CH₂); 3,41 - 3,48 (m, 1 H, N(C*H*₂)₂(CH₂)₂CH₂); 3,62 - 3,72 (m, 1 H, N(C*H*₂)₂(CH₂)₂CH₂); 3,84 (s, 3 H, NC*H*₃); 4,15 - 4,20 (m, 1 H, CH₃C*H*₂CO₂); 4,27 - 4,33 (m, 1 H, CH₃C*H*₂CO₂); 5,21 (d, 1 H, *J* = 5,3 Hz, C*H*N(CH₂)₂(CH₂)₂CH₂); 6,81 - 6,86 (m, 1 H, N(CH₃)]-CC*H*C*H*C-[); 7,08 - 7,14 (m, 1 H, N(CH₃)]-CC*H*C*H*C); 7,92 (d, 1 H, *J* = 7,5 Hz, NH); 10,22 (s, 1 H, HCl).

### Pharmakologische Untersuchungen:

### a) Affinität zum ORL-1-Rezeptor

Die Affinität der erfindungsgemäßen 5-Aminomethyl-1H-pyrrol-2-carbonsäureamide zum ORL-1-Rezeptor wurde wie vorstehend beschrieben bestimmt. Der Wert für eine ausgewählte Verbindung ist in der nachfolgenden Tabelle 4 wiedergegeben.

**Tabelle 4:**

| Verbindung gemäß Beispiel | ORL-1-human Inhibierung [%] |
|---|---|
| 9 | 23 |

### b) Affinität zum µ-Rezeptor

Die Affinität der erfindungsgemäßen 5-Aminomethyl-1H-pyrrol-2-carbonsäureamide zum µ-Rezeptor wurde wie vorstehend beschrieben bestimmt. Die Werte für einige ausgewählte Verbindungen ist in der nachfolgenden Tabelle 5 wiedergegeben.

**Tabelle 5:**

| Verbindung gemäß Beispiel | ORm-human Nal Inhibierung [%] | ORm-human Nal Ki [µM] |
|---|---|---|
| 1 | 62 | 0,16 |
| 2 | 49 | 0,36 |
| 4 | 80 | 0,79 |
| 5 | 62 | * |
| 7 | 65 | 1,0 |
| 9 | 76 | * |
| 11 | 33 | * |

| | | |
|---|---|---|
| * nicht bestimmt | | |

### c1) 5-HT-Uptake-Inhibierung

Die 5-HT-Uptake-Inhibierung der erfindungsgemäßen 5-Aminomethyl-1H-pyrrol-2-carbonsäureamide wurde wie vorstehend beschrieben bestimmt. Die Werte für einige ausgewählte Verbindungen sind in der nachfolgenden Tabelle 6 wiedergegeben.

**Tabelle 6:**

| Verbindung gemäß Beispiel | Uptake 5-HT-Inhibierung [%] conc. 10 |
|---|---|
| 1 | 57 |
| 2 | 52 |
| 4 | 72 |
| 5 | 57 |
| 6 | 51 |
| 7 | 93 |
| 8 | 79 |
| 11 | 54 |

### c2) Noradrenalin-Wiederaufnahmehemmung

Die Noradrenalin-Wiederaufnahmehemmung der erfindungsgemäßen 5-Aminomethyl-1H-pyrrol-2-carbonsäureamide wurde wie vorstehend beschrieben bestimmt. Die Werte für einige ausgewählte Verbindungen sind in der nachfolgenden Tabelle 7 wiedergegeben.

**Tabelle 7:**

| Verbindung gemäß Beispiel | Uptake Noradrenalin-Inhibierung [%] conc. 10 |
|---|---|
| 1 | 80 |
| 2 | 72 |
| 3 | 38 |
| 4 | 93 |
| 5 | 86 |
| 6 | 83 |
| 7 | 95 |
| 8 | 43 |
| 9 | 70 |
| 10 | 52 |

## Patentansprüche

1. Substituierte 5-Aminomethyl-1H-pyrrol-2-carbonsäureamide der allgemeinen Formel I, 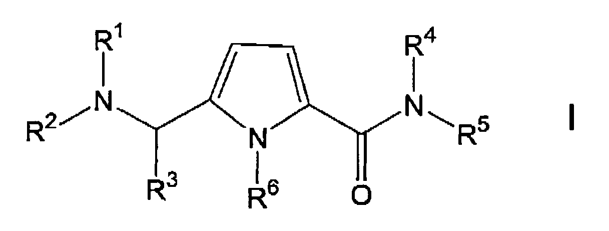 worin
R¹ für Wasserstoff, einen linearen oder verzweigten, gesättigten oder ungesättigten, ggf. wenigstens einfach substituierten aliphatischen C₁₋₈-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroaryl-Rest oder für einen über eine ggf. substituierte C₁₋₃-Alkylen-Gruppe gebundenen, ggf. wenigstens einfach substituierten, fünf- oder sechsgliedrigen Aryl- oder Heteroaryl-Rest steht,
R² für einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroaryl-Rest oder für einen über eine ggf. substituierte C₁₋₃-Alkylen-Gruppe gebundenen, ggf. wenigstens einfach substituierten, fünf- oder sechsgliedrigen Aryl- oder Heteroaryl-Rest steht, oder
R¹ und R² gemeinsam mit dem sie verbindenden Stickstoffatom als Ringglied einen gesättigten oder ungesättigten, ggf. wenigstens einfach substituierten, fünf- oder sechsgliedrigen, cycloaliphatischen Rest bilden, der ggf. wenigstens ein weiteres Heteroatom ausgewählt aus der Gruppe bestehend aus N, O und S als Ringglied aufweist,
R³ für einen ggf. wenigstens einfach substituierten Phenylrest oder eine Alkylester-Gruppe mit C₁₋₃ im Alkylteil steht,
R⁴ und R⁵, gleich oder verschieden, für Wasserstoff, einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten fünf- oder sechsgliedrigen Aryl- oder Heteroaryl-Rest oder für einen über eine ggf. substituierte C₁₋₃-Alkylen-Gruppe gebundenen, ggf. wenigstens einfach substituierten, fünf- oder sechsgliedrigen Aryl- oder Heteroaryl-Rest stehen, oder
R⁴ und R⁵ gemeinsam mit dem sie verbindenden Stickstoffatom als Ringglied einen gesättigten oder ungesättigten, ggf. wenigstens einfach substituierten fünf-, sechs- oder siebengliedrigen cycloaliphatischen Rest bilden, der ggf. wenigstens ein weiteres Heteroatom ausgewählt aus der Gruppe bestehend aus N, O und S als Ringglied aufweist, und
R⁶ für einen linearen oder verzweigten, gesättigten oder ungesättigten, ggf. wenigstens einfach substituierten aliphatischen C₁₋₆-Rest, einen ggf. über eine ggf. wenigstens einfach substituierte C₁₋₃-Alkylen-Gruppe gebundenen, gesättigten oder ungesättigten, ggf. wenigstens einfach substituierten, ggf. wenigstens ein Heteroatom als Ringglied aufweisenden cycloaliphatischen C₃₋₈-Rest, einen ggf. wenigstens einfach substituierten, fünf- oder sechsgliedrigen Aryl- oder Heteroaryl-Rest, einen über eine ggf. wenigstens einfach substituierte C₁₋₃-Alkylen-Gruppe gebundenen, ggf. wenigstens einfach substituierten fünf- oder sechsgliedrigen Aryl- oder Heteroaryl-Rest steht,
wobei
sofern einer der vorstehend genannten Reste R¹ bis R⁶ für einen aliphatischen oder cycloaliphatischen Rest steht, der einfach oder mehrfach substituiert ist, die Substituenten ausgewählt sind aus der Gruppe bestehend aus Halogen, Hydroxy, CN, CF₃, CHF₂, CH₂F, C₁₋₆-Alkyl, C₁₋₆-Alkoxy und ggf. wenigstens einfach substituiertem Phenyl, wobei sofern der Phenylsubstituent selbst einfach oder mehrfach substituiert ist, dessen Substituenten ausgewählt sind aus der Gruppe bestehend aus Hydroxy, CN, CF₃, CHF₂, CH₂F, C₁₋₆-Alkyl und C₁₋₆-Alkoxy,
sofern die vorstehend genannten Reste R¹ bis R⁶ eine einfach oder mehrfach substituierte Alkylen-Gruppe aufweisen, deren Substituenten ausgewählt sind aus der Gruppe bestehend aus Halogen, Hydroxy, CN, CF₃, CHF₂, CH₂F, C₁₋₆-Alkoxy und ggf. wenigstens einfach substituiertem Phenyl, wobei sofern der Phenylsubstituent selbst einfach oder mehrfach substituiert ist, dessen Substituenten ausgewählt sind aus der Gruppe bestehend aus Hydroxy, CN, CF₃, CHF₂, CH₂F, C₁₋₆-Alkyl und C₁₋₆-Alkoxy,
sofern einer der vorstehend genannten Reste R¹ bis R⁶ einen einfach oder mehrfach substituierten Aryl- oder Heteroaryl-Rest aufweist, die entsprechenden Substituenten ausgewählt sind aus der Gruppe bestehend aus Halogen, Hydroxy, CN, CF₃, CHF₂, CH₂F, NO₂, NH₂, C₁₋₆-Alkyl, C₁₋₆-Alkoxy und ggf. wenigstens einfach substituiertem Phenyl, wobei sofern der Phenylsubstituent selbst einfach oder mehrfach substituiert ist, dessen Substituenten ausgewählt sind aus der Gruppe bestehend aus Hydroxy, CN, CF₃, CHF₂, CH₂F, C₁₋₆-Alkyl und C₁₋₆-Alkoxy, und
sofern einer der vorstehend genannten Reste R¹ bis R⁶ für einen cycloaliphatischen Rest mit wenigstens einem Heteroatom oder einen Heteroarylrest steht, die Heteroatome ausgewählt sind aus der Gruppe bestehend aus Sauerstoff, Stickstoff und Schwefel,
gegebenenfalls in Form ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder ihrer Basen oder in Form ihrer Salze, insbesondere der physiologisch verträglichen Salze, oder jeweils in Form ihrer Solvate, insbesondere der Hydrate.

2. Substituierte 5-Aminomethyl-1H-pyrrol-2-carbonsäureamide gemäß Anspruch 1, **dadurch gekennzeichnet, daß**
R¹ für einen linearen oder verzweigten, gesättigten aliphatischen C₁₋₃-Rest, bevorzugt für eine Methylgruppe, steht.

3. Substituierte 5-Aminomethyl-1H-pyrrol-2-carbonsäureamide gemäß Anspruch 1 oder 2, **dadurch gekennzeichnet, daß**
R² für einen linearen oder verzweigten, gesättigten aliphatischen C₁₋₃-Rest, bevorzugt für eine Methylgruppe, steht.

4. Substituierte 5-Aminomethyl-1H-pyrrol-2-carbonsäureamide gemäß Anspruch 1, **dadurch gekennzeichnet, daß**
R¹ und R² gemeinsam mit dem sie verbindenden Stickstoffatom als Ringglied einen gesättigten, ggf. Sauerstoff als weiteres Ringglied aufweisenden, fünf-oder sechsgliedrigen cycloaliphatischen Rest bilden, bevorzugt gemeinsam für einen (CH₂)₄-, (cm₂)₅- oder (CH₂)₂-O-(CH₂)₂-Rest stehen, der mit dem sie verbindenden Stickstoffatom als Ringglied einen Heterocyclus bildet.

5. Substituierte 5-Aminomethyl-1H-pyrrol-2-carbonsäureamide gemäß einem der Ansprüche 1-4, **dadurch gekennzeichnet, daß**
R³ für einen ggf. wenigstens einfach substituierten Phenylrest oder eine Alkylester-Gruppe mit C₁₋₂ im Alkylteil steht.

6. Substituierte 5-Aminomethyl-1H-pyrrol-2-carbonsäureamide gemäß einem der Ansprüche 1-5, **dadurch gekennzeichnet, daß**
R⁴ und R⁵ gemeinsam mit dem sie verbindenden Stickstoffatom als Ringglied einen gesättigten, ggf. Sauerstoff als weiteres Ringglied aufweisenden, fünf-oder sechsgliedrigen cycloaliphatischen Rest bilden, bevorzugt gemeinsam für einen (NCH₂)₄-, (CH₂)₅- oder (CH₂)₂-O-(CH₂)₂-Rest stehen, der gemeinsam mit dem sie verbindenden Stickstoffatom als Ringglied einen Heterocyclus bildet.

7. Substituierte 5-Aminomethyl-1H-pyrrol-2-carbonsäureamide gemäß einem der Ansprüche 1-6, **dadurch gekennzeichnet, daß**
R⁶ für einen linearen oder verzweigten, gesättigten aliphatischen C₁₋₃-Rest, bevorzugt für einen Methylrest, steht.

8. Substituierte 5-Aminomethyl-1H-pyrrol-2-carbonsäureamide gemäß einem oder mehreren der Ansprüche 1-7 ausgewählt aus der Gruppe bestehend aus
5-[Dimethylamino-(4-fluoro-phenyl)-methyl]-1-methyl-1H-pyrrol-2-carbonsäurecyclohexylamid,
5-[Dimethylamino-(4-fluoro-phenyl)-methyl]-1-methyl-1H-pyrrol-2-carbonsäurebutylamid,
{5-[Dimethylamino-(4-fluoro-phenyl)-methyl]-1-methyl-1H-pyrrol-2-yl}pyrrolidin-1-yl-methanon,
5-[Dimethylamino-(4-bromo-phenyl)-methyl]-1-methyl-1H-pyrrol-2-carbonsäure-cyclohexylamid,
5-[Dimethylamino-(4-bromo-phenyl)-methyl]-1-methyl-1H-pyrrol-2-carbonsäure-butylamid,
{5-[(4-Bromo-phenyl)-dimethylamino-methyl]-1-methyl-1H-pyrrol-2-yl}-pyrrolidin-1-yl-methanon,
5-[(4-Bromo-phenyl)-dimethylamino-methyl]-1-methyl-1H-pyrrol-2-carbonsäure-phenethyl-amid,
{5-[(4-Bromo-phenyl)-dimethylamino-methyl]-1-methyl-1H-pyrrol-2-yl}-morpholin-4-yl-methanon,
5-(Dimethylamino-phenyl-methyl)-1-methyl-1H-pyrrol-2-carbonsäurecyclohexylamid,
5-(Dimethylamino-phenyl-methyl)-1-methyl-1H-pyrrol-2-carbonsäurebutylamid,
[5-(Dimethylamino-phenyl-methyl)-1-methyl-1H-pyrrol-2-yl]-pyrrolidin-1-yl-methanon,
(5-Butylcarbamoyl-1-methyl-1H-pyrrol-2-yl)-dimethylaminoessigsäureethylester,
(5-Cyclohexylcarbamoyl-1-methyl-1H-pyrrol-2-yl)-dimethylaminoessigsäureethylester,
(5-Butylcarbamoyl-1-methyl-1H-pyrrol-2-yl)-piperidin-1-yl-essigsäureethylester,
(5-Cyclohexylcarbamoyl-1-methyl-1H-pyrrol-2-yl)-piperidin-1-yl-essigsäureethylester,
gegebenenfalls in Form, ihrer reinen Stereoisomeren, inbesondere Enantiomeren oder Diastereomeren, ihrer Racemate oder in Form von Mischungen der Stereoisomeren, insbesondere der Enantiomeren, Diastereomeren oder Rotameren, in einem beliebigen Mischungsverhältnis, oder jeweils in Form ihrer Säuren oder ihrer Basen oder in Form ihrer Salze, insbesondere der physiologisch verträglichen Salze, besonders bevorzugt der Hydrochloride, oder jeweils in Form ihrer Solvate, insbesondere der Hydrate.

9. Verfahren zur Herstellung von substituierten 5-aminomethyl-1H-pyrrol-2-carbonsäureamiden gemäß einem der Ansprüche 1-8, **dadurch gekennzeichnet, daß** ein substituiertes 1H-pyrrol-2-carbonsäureamid der allgemeinen Formel II 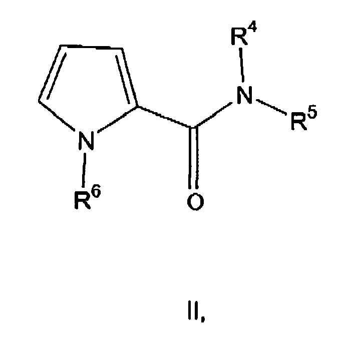 worin die Reste R⁴, R⁵ und R³ die Bedeutung gemäß Anspruch 1 haben mit einem Imminiumsalz der allgemeinen Formel III, 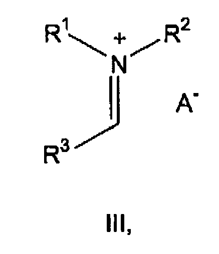 worin R¹ bis R³ die Bedeutung gemäß Anspruch 1 haben und A⁻ für ein geeignetes Anion, vorzugsweise für Cl⁻, AlCl₄⁻, Br⁻, I⁻ oder CF₃-SO₃⁻ (Triflat-Anion) steht, umgesetzt und ggf. gereinigt und ggf. isoliert wird.

10. Arzneimittel enthaltend wenigstens ein substituiertes 5-Aminomethyl-1H-pyrrol-2-carbonsäureamid gemäß einem der Ansprüche 1-8 sowie ggf. einen oder mehrere physiologisch verträgliche Hilfsstoffe.

11. Verwendung wenigstens eines substituierten 5-Aminomethyl-1H-pyrrol-2-carbonsäureamids gemäß einem der Ansprüche 1-8 zur Herstellung eines Arzneimittels zur Bekämpfung von Schmerzen.

12. Verwendung gemäß Anspruch 11 zur Bekämpfung von akuten Schmerzen.

13. Verwendung gemäß Anspruch 11 zur Bekämpfung von chronischen Schmerzen.

14. Verwendung gemäß Anspruch 11 zur Behandlung von neuropathischen Schmerzen.

15. Verwendung wenigstens eines substituierten 5-Aminomethyl-1H-pyrrol-2-carbonsäureamids gemäß einem der Ansprüche 1-8 zur Herstellung eines Arzneimittels zur Behandlung von Entzugserscheinungen.

16. Verwendung wenigstens eines substituierten 5-Aminomethyl-1H-pyrrol-2-carbonsäureamids gemäß einem der Ansprüche 1-8 zur Herstellung eines Arzneimittels zur Anxiolyse.

17. Verwendung wenigstens eines substituierten 5-Aminomethyl-1H-pyrroi-2-carbonsäureamids gemäß einem der Ansprüche 1-8 zur Herstellung eines Arzneimittels zur Behandlung von Gedächtnis-Störungen.

18. Verwendung wenigstens eines substituierten 5-Aminomethyl-1H-pyrrol-2-carbonsäureamids gemäß einem der Ansprüche 1-8 zur Herstellung eines Arzneimittels zur Behandlung von neurodegenerativen Erkrankungen, vorzugsweise Morbus Parkinson, Morbus Huntington und/oder Morbus Alzheimer.

19. Verwendung wenigstens eines substituierten 5-Aminomethyl-1H-pyrrol-2-carbonsäureamids gemäß einem der Ansprüche 1-8 zur Herstellung eines Arzneimittels zur Behandlung von Epilepsie.

20. Verwendung wenigstens eines substituierten 5-Aminomethyl-1H-pyrrol-2-carbonsäureamids gemäß einem der Ansprüche 1-8 zur Herstellung eines Arzneimittels zur Diurese.

21. Verwendung wenigstens eines substituierten 5-Aminomethyl-1H-pyrrol-2-carbonsäureamids gemäß einem der Ansprüche 1-8 zur Herstellung eines Arzneimittels zur Behandlung cardiovaskulärer Störungen.

22. Verwendung wenigstens eines substituierten 5-Aminomethyl-1H-pyrrol-2-carbonsäureamids gemäß einem der Ansprüche 1-8 zur Herstellung eines Arzneimittels zur Behandlung von Wasserspeicher-Krankheiten.

23. Verwendung wenigstens eines substituierten 5-Aminomethyl-1H-pyrrol-2-carbonsäureamids gemäß einem der Ansprüche 1-8 zur Herstellung eines Arzneimittels zur Behandlung von intestinaler Motilität (Diarrhoe).

24. Verwendung wenigstens eines substituierten 5-Aminomethyl-1H-pyrrol-2-carbonsäureamids gemäß einem der Ansprüche 1-8 zur Herstellung eines Arzneimittels zur Unterdrückung des Miktionsreflexes.

25. Verwendung wenigstens eines substituierten 5-Aminomethyl-1H-pyrrol-2-carbonsäureamids gemäß einem der Ansprüche 1-8 zur Herstellung eines Arzneimittels zur Behandlung von Harninkontinenz.

26. Verwendung wenigstens eines substituierten 5-Aminomethyl-1H-pyrrol-2-carbonsäureamids gemäß einem der Ansprüche 1-8 zur Herstellung eines Arzneimittels zur Behandlung von Anorexie.

27. Verwendung wenigstens eines substituierten 5-Aminomethyl-1H-pyrrol-2-carbonsäureamids gemäß einem der Ansprüche 1-8 zur Herstellung eines Arzneimittels zur Behandlung von Tinnitus.

28. Verwendung wenigstens eines substituierten 5-Aminomethyl-1H-pyrrol-2-carbonsäureamids gemäß einem der Ansprüche 1-8 zur Herstellung eines Arzneimittels zur Behandlung von Pruritus.

29. Verwendung wenigstens eines substituierten 5-Aminomethyl-1H-pyrrol-2-carbonsäureamids gemäß einem der Ansprüche 1-8 zur Herstellung eines Arzneimittels zur Behandlung von Depressionen.

30. Verwendung wenigstens eines substituierten 5-Aminomethyl-1H-pyrrol-2-carbonsäureamids gemäß einem der Ansprüche 1-8 zur Herstellung eines Arzneimittels zur Regulation, vorzugsweise Stimulation, der Nahrungsaufnahme.

31. Verwendung wenigstens eines substituierten 5-Aminomethyl-1H-pyrrol-2-carbonsäureamids gemäß einem der Ansprüche 1-8 zur Herstellung eines Arzneimittels zur Reduzierung des Suchtpotentials von Opioiden, vorzugsweise von Morphin.

32. Verwendung wenigstens eines substituierten 5-Aminomethyl-1H-pyrrol-2-carbonsäureamids gemäß einem der Ansprüche 1-8 zur Herstellung eines Arzneimittels zur Modulation der Bewegungsaktivität.

33. Verwendung wenigstens eines substituierten 5-Aminomethyl-1H-pyrrol-2-carbonsäureamids gemäß einem der Ansprüche 1-8 zur Herstellung eines Arzneimittels zur Beeinflußung des cardiovaskulären Systems, vorzugsweise zur Vasodilatation der Arterien.

34. Verwendung wenigstens eines substituierten 5-Aminomethyl-1H-pyrrol-2-carbonsäureamids gemäß einem der Ansprüche 1-8 zur Herstellung eines Arzneimittels zur Behandlung sexueller Dysfunktionen, vorzugsweise erektiler Dysfunktion.

35. Verwendung wenigstens eines substituierten 5-Aminomethyl-1H-pyrrol-2-carbonsäureamids gemäß einem der Ansprüche 1-8 zur Herstellung eines Arzneimittels zur Behandlung von Atemwegserkrankungen.

36. Verwendung wenigstens eines substituierten 5-Aminomethyl-1H-pyrrol-2-carbonsäureamids gemäß einem der Ansprüche 1-8 zur Herstellung eines Arzneimittels zur Regulation des Elektrolyt-Haushaltes.

## Claims

1. Substituted 5-aminomethyl-1H-pyrrole-2-carboxylic acid amides of the general formula I, 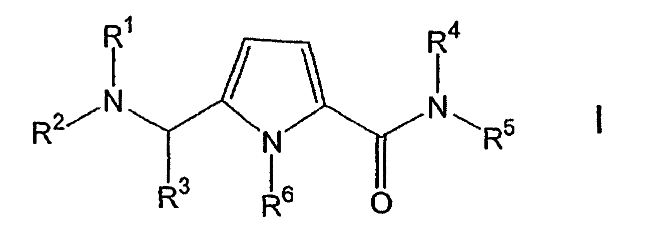 in which
R¹ denotes hydrogen, a linear or branched, saturated or unsaturated, optionally at least mono-substituted aliphatic C₁₋₆ residue, a saturated or unsaturated, optionally at least monosubstituted cycloaliphatic C₃₋₈ residue optionally comprising at least one heteroatom as a ring member and optionally attached via an optionally at least mono-substituted C₁₋₃ alkylene group, an optionally at least mono-substituted, five-or six-membered aryl or heteroaryl residue or an optionally at least mono-substituted, five- or six-membered aryl or heteroaryl residue attached via an optionally substituted C₁₋₃ alkylene group,
R² denotes a linear or branched, saturated or unsaturated, optionally at least mono-substituted aliphatic C₁₋₆ residue, a saturated or unsaturated, optionally at least monosubstituted cycloaliphatic C₃₋₈ residue optionally comprising at least one heteroatom as a ring member and optionally attached via an optionally at least mono-substituted C₁₋₃ alkylene group, an optionally at least mono-substituted, five-or six-membered aryl or heteroaryl residue or an optionally at least mono-substituted, five- or six-membered aryl or heteroaryl residue attached via an optionally substituted C₁₋₃ alkylene group,
R¹ and R², together with the nitrogen atom joining them as a ring member, form a saturated or unsaturated, optionally at least mono-substituted, five- or six-membered cycloaliphatic residue, which optionally comprises at least one further heteroatom selected from the group consisting of N, O and S as a ring member,
R³ denotes an optionally at least mono-substituted phenyl residue or an alkyl ester group with C₁₋₃ in the alkyl moiety,
R⁴ and R⁵, identical or different, denote hydrogen, a linear or branched, saturated or unsaturated, optionally at least mono-substituted aliphatic C₁₋₆ residue, a saturated or unsaturated, optionally at least monosubstituted cycloaliphatic C₃₋₈ residue optionally comprising at least one heteroatom as a ring member and optionally attached via an optionally at least mono-substituted C₁₋₃ alkylene group, an optionally at least mono-substituted, five- or six-membered aryl or heteroaryl residue or an optionally at least mono-substituted, five- or six-membered aryl or heteroaryl residue attached via an optionally substituted C₁₋₃ alkylene group,
R⁴ and R⁵, together with the nitrogen atom joining them as a ring member, form a saturated or unsaturated, optionally at least mono-substituted, five-, six- or seven-membered cycloaliphatic residue, which optionally comprises at least one further heteroatom selected from the group consisting of N, O and S as a ring member,
R⁶ denotes a linear or branched, saturated or unsaturated, optionally at least mono-substituted aliphatic C₁₋₆ residue, a saturated or unsaturated, optionally at least monosubstituted cycloaliphatic C₃₋₈ residue optionally comprising at least one heteroatom as a ring member and optionally attached via an optionally at least mono-substituted C₁₋₃ alkylene group, an optionally at least mono-substituted, five-or six-membered aryl or heteroaryl residue, an optionally at least mono-substituted, five- or six-membered aryl or heteroaryl residue attached via an optionally at least mono-substituted C₁₋₃ alkylene group,
wherein
if one of the above-stated residues R¹ to R⁶ denotes an aliphatic or cycloaliphatic residue which is mono-or polysubstituted, the substituents are selected from the group consisting of halogen, hydroxy, CN, CF₃, CHF₂, CH₂F, C₁₋₆ alkyl, C₁₋₆ alkoxy and optionally at least mono-substituted phenyl, wherein if the phenyl substituent is itself mono- or polysubstituted, the substituents thereof are selected from the group consisting of hydroxy, CN, CF₃, CHF₂, CH₂F, C₁₋₆ alkyl and C₁₋₆ alkoxy,
if the above-stated residues R¹ to R⁶ comprise a mono-or polysubstituted alkylene group, the substituents thereof are selected from the group consisting of halogen, hydroxy, CN, CF₃, CHF₂, CH₂F, C₁₋₆ alkoxy and optionally at least mono-substituted phenyl, wherein if the phenyl substituent is itself mono- or polysubstituted, the substituents thereof are selected from the group consisting of hydroxy, CN, CF₃, CHF₂, CH₂F, C₁₋₆ alkyl and C₁₋₆ alkoxy,
if one of the above-stated residues R¹ to R⁶ comprises a mono- or polysubstituted aryl or heteroaryl residue, the corresponding substituents are selected from the group consisting of halogen, hydroxy, CN, CF₃, CHF₂, CH₂F, NO₂, NH₂, C₁₋₆ alkyl, C₁₋₆ alkoxy and optionally at least mono-substituted phenyl, wherein if the phenyl substituent is itself mono- or polysubstituted, the substituents thereof are selected from the group consisting of hydroxy, CN, CF₃, CHF₂, CH₂F, C₁₋₆ alkyl and C₁₋₆ alkoxy, and
if one of the above-stated residues R¹ to R⁶ denotes a cycloaliphatic residue with at least one heteroatom or a heteroaryl residue, the heteroatoms are selected from the group consisting of oxygen, nitrogen and sulfur,
optionally in the form of the pure stereoisomers thereof, in particular enantiomers or diastereomers, the racemates thereof or in the form of mixtures of the stereoisomers, in particular the enantiomers, diastereomers or rotamers, in any desired mixing ratio, or in each case in the form of the acids or bases thereof or in the form of the salts thereof, in particular the physiologically acceptable salts, or in each case in the form of the solvates thereof, in particular the hydrates.

2. Substituted 5-aminomethyl-1H-pyrrole-2-carboxylic acid amides according to claim 1, **characterised in that**
R¹ denotes a linear or branched, saturated aliphatic C₁₋₃ residue, preferably a methyl group.

3. Substituted 5-aminomethyl-1H-pyrrole-2-carboxylic acid amides according to claim 1 or claim 2, **characterised in that**
R² denotes a linear or branched, saturated aliphatic C₁₋₃ residue, preferably a methyl group.

4. Substituted 5-aminomethyl-1H-pyrrole-2-carboxylic acid amides according to claim 1, **characterised in that**
R¹ and R², together with the nitrogen atom joining them as a ring member, form a saturated, five- or six-membered cycloaliphatic residue optionally comprising oxygen as a further ring member, preferably together denote a (CH₂)₄, (CH₂) or (CH₂)₂-O-(CH₂) residue which, with the nitrogen atom joining them as a ring member, forms a heterocycle.

5. Substituted 5-aminomethyl-1H-pyrrole-2-carboxylic acid amides according to any one of claims 1-4, **characterised in that**
R³ denotes an optionally at least mono-substituted phenyl residue or an alkyl ester group with C₁₋₂ in the alkyl moiety.

6. Substituted 5-aminomethyl-1H-pyrrole-2-carboxylic acid amides according to any one of claims 1-5, **characterised in that**
R⁴ and R⁵, together with the nitrogen atom joining them as a ring member, form a saturated, five- or six-membered cycloaliphatic residue optionally comprising oxygen as a further ring member, preferably together denote a (CH₂)₄, (CH₂) or (CH₂)₂-O-(CH₂)₂ residue which, together with the nitrogen atom joining them as a ring member, forms a heterocycle.

7. Substituted 5-aminomethyl-1H-pyrrole-2-carboxylic acid amides according to any one of claims 1-6, **characterised in that**
R⁶ denotes a linear or branched, saturated aliphatic C₁₋₃ residue, preferably a methyl residue.

8. Substituted 5-aminomethyl-1H-pyrrole-2-carboxylic acid amides according to one or more of claims 1-7 selected from the group consisting of
5-[dimethylamino-(4-fluorophenyl)-methyl]-1-methyl-1H-pyrrole-2-carboxylic acid cyclohexylamide,
5-[dimethylamino-(4-fluorophenyl)-methyl]-1-methyl-1H-pyrrole-2-carboxylic acid butylamide,
{5-[dimethylamino-(4-fluorophenyl)-methyl]-1-methyl-1H-pyrrol-2-yl}-pyrrolidin-1-yl-methanone,
5-[dimethylamino-(4-bromophenyl)-methyl]-1-methyl-1H-pyrrole-2-carboxylic acid cyclohexylamide,
5-[dimethylamino-(4-bromophenyl)-methyl]-1-methyl-1H-pyrrole-2-carboxylic acid butylamide,
{5-[(4-bromophenyl)-dimethylamino-methyl]-1-methyl-1H-pyrrol-2-yl}-pyrrolidin-1-yl-methanone,
5-[(4-bromophenyl)-dimethylamino-methyl]-1-methyl-1H-pyrrole-2-carboxylic acid phenethylamide,
{5-[(4-bromophenyl)-dimethylamino-methyl]-1-methyl-1H-pyrrol-2-yl}-morpholin-4-yl-methanone,
5-(dimethylaminophenylmethyl)-1-methyl-1H-pyrrole-2-carboxylic acid cyclohexylamide,
5-(dimethylaminophenylmethyl)-1-methyl-1H-pyrrole-2-carboxylic acid butylamide,
[5-(dimethylaminophenylmethyl)-1-methyl-1H-pyrrol-2-yl]-pyrrolidin-1-yl-methanone,
(5-butylcarbamoyl-1-methyl-1H-pyrrol-2-yl)-dimethylamino-acetic acid ethyl ester,
(5-cyclohexylcarbamoyl-1-methyl-1H-pyrrol-2-yl)-dimethylamino-acetic acid ethyl ester, (5-butylcarbamoyl-1-methyl-1H-pyrrol-2-yl)-piperidin-1-yl-acetic acid ethyl ester,
(5-cyclohexylcarbamoyl-1-methyl-1H-pyrrol-2-yl)-piperidin-1-yl-acetic acid ethyl ester,
optionally in the form of the pure stereoisomers thereof, in particular enantiomers or diastereomers, the racemates thereof or in the form of mixtures of the stereoisomers, in particular the enantiomers, diastereomers or rotamers, in any desired mixing ratio, or in each case in the form of the acids or bases thereof or in the form of the salts thereof, in particular the physiologically acceptable salts, particularly preferably the hydrochlorides, or in each case in the form of the solvates thereof, in particular the hydrates.

9. A process for the production of substituted 5-aminomethyl-1H-pyrrole-2-carboxylic acid amides according to any one of claims 1-8, **characterised in that** a substituted 1H-pyrrole-2-carboxylic acid amide of the general formula II 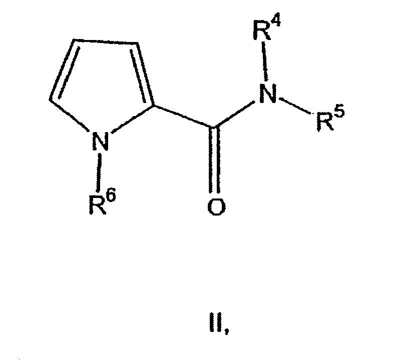 in which the residues R⁴, R⁵ and R⁶ have the meaning according to claim 1, is reacted with an iminium salt of the general formula III, 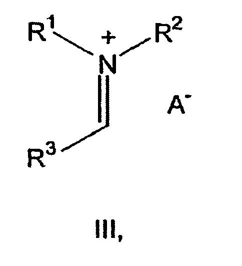 in which R¹ to R³ have the meaning according to claim 1 and A⁻ denotes a suitable anion, preferably Cl⁻, AlCl₄⁻, Br⁻, I⁻ or CF₃-SO₃⁻ (triflate anion) and is optionally purified and optionally isolated.

10. A medicament containing at least one substituted 5-aminomethyl-1H-pyrrole-2-carboxylic acid amide according to any one of claims 1-8 and optionally one or more physiologically acceptable auxiliary substances.

11. Use of at least one substituted 5-aminomethyl-1H-pyrrole-2-carboxylic acid amide according to any one of claims 1-8 for producing a medicament for combatting pain.

12. Use according to claim 11 for combatting acute pain.

13. Use according to claim 11 for combatting chronic pain.

14. Use according to claim 11 for the treatment of neuropathic pain.

15. Use of at least one substituted 5-aminomethyl-1H-pyrrole-2-carboxylic acid amide according to any one of claims 1-8 for producing a medicament for the treatment of withdrawal symptoms.

16. Use of at least one substituted 5-aminomethyl-1H-pyrrole-2-carboxylic acid amide according to any one of claims 1-8 for producing a medicament for anxiolysis.

17. Use of at least one substituted 5-aminomethyl-1H-pyrrole-2-carboxylic acid amide according to any one of claims 1-8 for producing a medicament for the treatment of memory disorders.

18. Use of at least one substituted 5-aminomethyl-1H-pyrrole-2-carboxylic acid amide according to any one of claims 1-8 for producing a medicament for the treatment of neurodegenerative diseases, preferably Parkinson's disease, Huntington's chorea and/or Alzheimer's disease.

19. Use of at least one substituted 5-aminomethyl-1H-pyrrole-2-carboxylic acid amide according to any one of claims 1-8 for producing a medicament for the treatment of epilepsy.

20. Use of at least one substituted 5-aminomethyl-1H-pyrrole-2-carboxylic acid amide according to any one of claims 1-8 for producing a medicament for diuresis.

21. Use of at least one substituted 5-aminomethyl-1H-pyrrole-2-carboxylic acid amide according to any one of claims 1-8 for producing a medicament for the treatment of cardiovascular disorders.

22. Use of at least one substituted 5-aminomethyl-1H-pyrrole-2-carboxylic acid amide according to any one of claims 1-8 for producing a medicament for the treatment of water retention conditions.

23. Use of at least one substituted 5-aminomethyl-1H-pyrrole-2-carboxylic acid amide according to any one of claims 1-8 for producing a medicament for the treatment of intestinal motility (diarrhoea).

24. Use of at least one substituted 5-aminomethyl-1H-pyrrole-2-carboxylic acid amide according to any one of claims 1-8 for producing a medicament for the suppression of the urinary reflex.

25. Use of at least one substituted 5-aminomethyl-1H-pyrrole-2-carboxylic acid amide according to any one of claims 1-8 for producing a medicament for the treatment of urinary incontinence.

26. Use of at least one substituted 5-aminomethyl-1H-pyrrole-2-carboxylic acid amide according to any one of claims 1-8 for producing a medicament for the treatment of anorexia.

27. Use of at least one substituted 5-aminomethyl-1H-pyrrole-2-carboxylic acid amide according to any one of claims 1-8 for producing a medicament for the treatment of tinnitus.

28. Use of at least one substituted 5-aminomethyl-1H-pyrrole-2-carboxylic acid amide according to any one of claims 1-8 for producing a medicament for the treatment of pruritus.

29. Use of at least one substituted 5-aminomethyl-1H-pyrrole-2-carboxylic acid amide according to any one of claims 1-8 for producing a medicament for the treatment of depression.

30. Use of at least one substituted 5-aminomethyl-1H-pyrrole-2-carboxylic acid amide according to any one of claims 1-8 for producing a medicament for regulating, preferably stimulating, food intake.

31. Use of at least one substituted 5-aminomethyl-1H-pyrrole-2-carboxylic acid amide according to any one of claims 1-8 for producing a medicament for reducing the addictive potential of opioids, preferably of morphine.

32. Use of at least one substituted 5-aminomethyl-1H-pyrrole-2-carboxylic acid amide according to any one of claims 1-8 for producing a medicament for modulating locomotor activity.

33. Use of at least one substituted 5-aminomethyl-1H-pyrrole-2-carboxylic acid amide according to any one of claims 1-8 for producing a medicament for influencing the cardiovascular system, preferably for vasodilating the arteries.

34. Use of at least one substituted 5-aminomethyl-1H-pyrrole-2-carboxylic acid amide according to any one of claims 1-8 for producing a medicament for treating sexual dysfunction, preferably erectile dysfunction.

35. Use of at least one substituted 5-aminomethyl-1H-pyrrole-2-carboxylic acid amide according to any one of claims 1-8 for producing a medicament for the treatment of airways diseases.

36. Use of at least one substituted 5-aminomethyl-1H-pyrrole-2-carboxylic acid amide according to any one of claims 1-8 for producing a medicament for regulating the electrolyte balance.

## Revendications

1. Amides substitués de l'acide 5-aminométhyl-1H-pyrrole-2-carboxylique de formule générale I, 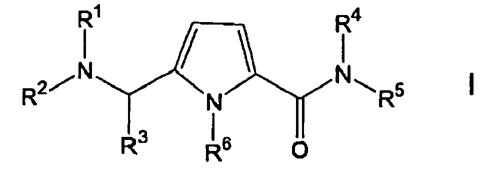 où
R¹ représente hydrogène, un radical C₁₋₆ aliphatique, linéaire ou ramifié, saturé ou insaturé, le cas échéant au moins monosubstitué, un radical C₃₋₈ cycloaliphatique, saturé ou insaturé, le cas échéant au moins monosubstitué, présentant le cas échéant au moins un hétéroatome comme élément de cycle, le cas échéant lié via un groupe C₁₋₃-alkylène le cas échéant au moins monosubstitué, un radical aryle ou hétéroaryle de cinq ou six chaînons, le cas échéant au moins monosubstitué ou un radical aryle ou hétéroaryle de cinq ou six chaînons, le cas échéant au moins monosubstitué, lié via un groupe C₁₋₃-alkylène le cas échéant substitué,
R² représente un radical C₁₋₆ aliphatique, linéaire ou ramifié, saturé ou insaturé, le cas échéant au moins monosubstitué, un radical C₃₋₈ cycloaliphatique, saturé ou insaturé, le cas échéant au moins monosubstitué, présentant le cas échéant au moins un hétéroatome comme élément de cycle, le cas échéant lié via un groupe C₁₋₃-alkylène le cas échéant au moins monosubstitué, un radical aryle ou hétéroaryle de cinq ou six chaînons, le cas échéant au moins monosubstitué ou un radical aryle ou hétéroaryle de cinq ou six chaînons, le cas échéant au moins monosubstitué, lié via un groupe C₁₋₃-alkylène le cas échéant substitué, ou
R¹ et R² formant, ensemble avec l'atome d'azote qui les relie comme élément de cycle un radical cycloaliphatique de cinq ou six chaînons saturé ou insaturé, le cas échéant au moins monosubstitué, qui présente le cas échéant au moins un autre hétéroatome choisi dans le groupe constitué par N, O et S comme élément de cycle,
R³ représente un radical phényle le cas échéant au moins monosubstitué ou un groupe ester d'alkyle en C₁₋₃ dans la partie alkyle,
R⁴ et R⁵ sont identiques ou différents et représentent hydrogène, un radical C₁₋₆ aliphatique, linéaire ou ramifié, saturé ou insaturé, le cas échéant au moins monosubstitué, un radical C₃₋₈ cycloaliphatique, saturé ou insaturé, le cas échéant au moins monosubstitué, présentant le cas échéant au moins un hétéroatome comme élément de cycle, le cas échéant lié via un groupe C₁₋₃-alkylène le cas échéant au moins monosubstitué, un radical aryle ou hétéroaryle de cinq ou six chaînons, le cas échéant au moins monosubstitué ou un radical aryle ou hétéroaryle de cinq ou six chaînons, le cas échéant au moins monosubstitué, lié via un groupe C₁₋₃-alkylène le cas échéant substitué, ou
R⁴ et R⁵ forment, ensemble avec l'atome d'azote qui les relie comme élément de cycle un radical cycloaliphatique de cinq ou six chaînons saturé ou insaturé, le cas échéant au moins monosubstitué, qui présente le cas échéant au moins un autre hétéroatome choisi dans le groupe constitué par N, O et S comme élément de cycle, et
R⁶ représente un radical C₁₋₆ aliphatique, linéaire ou ramifié, saturé ou insaturé, le cas échéant au moins monosubstitué, un radical C₃₋₈ cycloaliphatique, saturé ou insaturé, le cas échéant au moins monosubstitué, présentant le cas échéant au moins un hétéroatome comme élément de cycle, le cas échéant lié via un groupe C₁₋₃-alkylène le cas échéant au moins monosubstitué, un radical aryle ou hétéroaryle de cinq ou six chaînons, le cas échéant au moins monosubstitué ou un radical aryle ou hétéroaryle de cinq ou six chaînons, le cas échéant au moins monosubstitué, lié via un groupe C₁₋₃-alkylène le cas échéant au moins monosubstitué,
où
- pour autant qu'un des radicaux R¹ à R⁶ susmentionnés représente un radical aliphatique ou cycloaliphatique, qui est monosubstitué ou polysubstitué, les substituants sont choisis dans le groupe constitué par halogène, hydroxy, CN, CF₃, CHF₂, CH₂F, C₁₋₆ -alkyle, C₁₋₆-alcoxy et phényle le cas échéant au moins monosubstitué, où, pour autant que le substituant phényle soit lui-même monosubstitué ou polysubstitué, ses substituants sont choisis dans le groupe constitué par hydroxy, CN, CF₃, CHF₂, CH₂F, C₁₋₆-alkyle et C₁₋₆-alcoxy,
- pour autant qu'un des radicaux R¹ à R⁶ susmentionnés présente un groupe alkylène monosubstitué ou polysubstitué, leurs substituants sont choisis dans le groupe constitué par halogène, hydroxy, CN, CF₃, CHF₂, CH₂F, C₁₋₆-alcoxy et phényle le cas échéant au moins monosubstitué, où, pour autant que le substituant phényle soit lui-même monosubstitué ou polysubstitué, ses substituants sont choisis dans le groupe constitué par hydroxy, CN, CF₃, CHF₂, CH₂F, C₁₋₆-alkyle et C₁₋₆-alcoxy,
- pour autant qu'un des radicaux R¹ à R⁶ susmentionnés présente un radical aryle ou hétéroaryle monosubstitué ou polysubstitué, les substituants correspondants sont choisis dans le groupe constitué par halogène, hydroxy, CN, CF₃, CHF₂, CH₂F, NO₂, NH₂, C₁₋₆-alkyle, C₁₋₆-alcoxy et phényle le cas échéant au moins monosubstitué, où, pour autant que le substituant phényle soit lui-même monosubstitué ou polysubstitué, ses substituants sont choisis dans le groupe constitué par hydroxy, CN, CF₃, CHF₂, CH₂F, C₁₋₆-alkyle et C₁₋₆-alcoxy, et
- pour autant qu'un des radicaux R¹ à R⁶ susmentionnés représente un radical cycloaliphatique comprenant au moins un hétéroatome ou un radical hétéroaryle, les hétéroatomes sont choisis dans le groupe constitué par oxygène, azote et soufre,
le cas échéant sous forme de leurs stéréo-isomères purs, en particulier les énantiomères ou diastéréo-isomères, leurs racémates ou sous forme de mélanges des stéréo-isomères, en particulier des énantiomères, des diastéréo-isomères ou des rotamères, dans un rapport de mélange quelconque ou à chaque fois sous forme de leurs acides ou de leurs bases ou sous forme de leurs sels, en particulier des sels physiologiquement acceptables, ou à chaque fois sous forme de leurs solvates, en particulier les hydrates.

2. Amides substitués de l'acide 5-aminométhyl-1H-pyrrole-2-carboxylique selon la revendication 1, **caractérisés en ce que**
R¹ représente un radical aliphatique en C₁₋₃ linéaire ou ramifié, saturé, de préférence un groupe méthyle.

3. Amides substitués de l'acide 5-aminométhyl-1H-pyrrole-2-carboxylique selon la revendication 1 ou 2, **caractérisés en ce que**
R² représente un radical aliphatique en C₁₋₃ linéaire ou ramifié, saturé, de préférence un groupe méthyle.

4. Amides substitués de l'acide 5-aminométhyl-1H-pyrrole-2-carboxylique selon la revendication 1, **caractérisés en ce que**
R¹ et R² forment, ensemble avec l'atome d'azote qui les relie comme élément de cycle, un radical cycloaliphatique de cinq ou six chaînons, saturé, présentant le cas échéant de l'oxygène comme autre élément de cycle, de préférence ensemble un radical (CH₂)₄, (CH₂)₅ ou (CH₂)₂-O-(CH₂)₂, qui forme, avec l'atome d'azote qui les relie comme élément de cycle, un hétérocycle.

5. Amides substitués de l'acide 5-aminométhyl-1H-pyrrole-2-carboxylique selon l'une quelconque des revendications 1 à 4, **caractérisés en ce que**
R³ représente un radical phényle le cas échéant au moins monosubstitué ou un groupe ester d'alkyle en C₁₋₂ dans la partie alkyle.

6. Amides substitués de l'acide 5-aminométhyl-1H-pyrrole-2-carboxylique selon l'une quelconque des revendications 1 à 5, **caractérisés en ce que**
R⁴ et R⁵ forment, ensemble avec l'atome d'azote qui les relie comme élément de cycle, un radical cycloaliphatique de cinq ou six chaînons, saturé, présentant le cas échéant de l'oxygène comme autre élément de cycle, de préférence ensemble un radical (CH₂)₄, (CH₂)₅ ou (CH₂)₂-O-(CH₂)₂, qui forme, avec l'atome d'azote qui les relie comme élément de cycle, un hétérocycle.

7. Amides substitués de l'acide 5-aminométhyl-1H-pyrrole-2-carboxylique selon l'une quelconque des revendications 1 à 6, **caractérisés en ce que**
R⁶ représente un radical aliphatique en C₁₋₃ linéaire ou ramifié, saturé, de préférence un radical méthyle.

8. Amides substitués de l'acide 5-aminométhyl-1H-pyrrole-2-carboxylique selon l'une ou plusieurs des revendications 1 à 7, choisis dans le groupe constitué par
- le cyclohexylamide de l'acide 5-[diméthylamino-(4-fluorophényl)-méthyl]-1-méthyl-1H-pyrrole-2-carboxylique,
- le butylamide de l'acide 5-[diméthylamino-(4-fluorophényl)-méthyl]-1-méthyl-1H-pyrrole-2-carboxylique,
- la {5-[diméthylamino-(4-fluorophényl)-méthyl]-1-méthyl-1H-pyrrol-2-yl}-pyrrolidin-1-ylméthanone,
- le cyclohexylamide de l'acide 5-[diméthylamino-(4-bromophényl)-méthyl]-1-méthyl-1H-pyrrole-2-carboxylique,
- le butylamide de l'acide 5-[diméthylamino-(4-bromophényl)-méthyl]-1-méthyl-1H-pyrrole-2-carboxylique,
- la {5-[(4-bromophényl)-diméthylaminométhyl]-1-méthyl-1H-pyrrol-2-yl}-pyrrolidin-1-ylméthanone,
- le phénéthylamide de l'acide 5-[(4-bromophényl)-diméthylaminométhyl]-1-méthyl-1H-pyrrole-2-carboxylique,
- la {5-[(4-bromophényl)-diméthylaminométhyl]-1-méthyl-1H-pyrrol-2-yl}-morpholin-4-ylméthanone,
- le cyclohexylamide de l'acide 5-(diméthylaminophénylméthyl)-1-méthyl-1H-pyrrole-2-carboxylique,
- le butylamide de l'acide 5-(diméthylaminophénylméthyl)-1-méthyl-1H-pyrrole-2-carboxylique,
- la [5-(diméthylaminophénylméthyl)-1-méthyl-1H-pyrrol-2-yl]-pyrrolidin-1-ylméthanone,
- l'ester éthylique de l'acide (5-butylcarbamoyl-1-méthyl-1H-pyrrol-2-yl)-diméthylaminoacétique,
- l'ester éthylique de l'acide (5-cyclohexylcarbamoyl-1-méthyl-1H-pyrrol-2-yl)-diméthylaminoacétique,
- l'ester éthylique de l'acide (5-butylcarbamoyl-1-méthyl-1H-pyrrol-2-yl)-pipéridin-1-ylacétique,
- l'ester éthylique de l'acide (5-cyclohexylcarbamoyl-1-méthyl-1H-pyrrol-2-yl)-pipéridin-1-ylacétique,
le cas échéant sous forme de leurs stéréo-isomères purs, en particulier les énantiomères ou diastéréo-isomères, leurs racémates ou sous forme de mélanges des stéréo-isomères, en particulier des énantiomères, des diastéréo-isomères ou des rotamères, dans un rapport de mélange quelconque ou à chaque fois sous forme de leurs acides ou de leurs bases ou sous forme de leurs sels, en particulier des sels physiologiquement acceptables, de manière particulièrement préférée les chlorhydrates, ou à chaque fois sous forme de leurs solvates, en particulier les hydrates.

9. Procédé pour la préparation d'amides substitués de l'acide 5-aminométhyl-1H-pyrrole-2-carboxylique selon l'une quelconque des revendications 1 à 8, **caractérisé en ce qu'**on transforme un amide substitué de l'acide 1H-pyrrole-2-carboxylique de formule générale II, 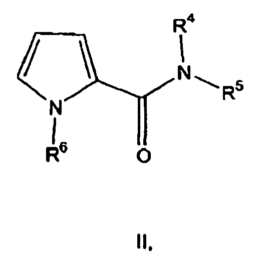 où les radicaux R⁴, R⁵ et R⁶ présentent la signification selon la revendication 1, avec un sel d'iminium de formule générale III, 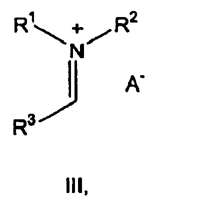 où R¹ à R³ présentent la signification selon la revendication 1 et A⁻ représente un anion approprié, de préférence Cl⁻, AlCl₄⁻, Br⁻, I⁻ ou CF₃-SO₃⁻ (anion triflate), on purifie le cas échéant et on isole le cas échéant.

10. Médicament contenant au moins un amide substitué de l'acide 5-aminométhyl-1H-pyrrole-2-carboxylique selon l'une quelconque des revendications 1 à 8 ainsi que le cas échéant un ou plusieurs adjuvants physiologiquement acceptables.

11. Utilisation d'au moins un amide substitué de l'acide 5-aminométhyl-1H-pyrrole-2-carboxylique selon l'une quelconque des revendications 1 à 8 pour la préparation d'un médicament destiné à lutter contre les douleurs.

12. Utilisation selon la revendication 11 pour lutter contre des douleurs aiguës.

13. Utilisation selon la revendication 11 pour lutter contre des douleurs chroniques.

14. Utilisation selon la revendication 11 pour le traitement de douleurs neuropathiques.

15. Utilisation d'au moins un amide substitué de l'acide 5-aminométhyl-1H-pyrrole-2-carboxylique selon l'une quelconque des revendications 1 à 8 pour la préparation d'un médicament destiné au traitement de symptômes de sevrage.

16. Utilisation d'au moins un amide substitué de l'acide 5-aminométhyl-1H-pyrrole-2-carboxylique selon l'une quelconque des revendications 1 à 8 pour la préparation d'un médicament destiné à l'anxiolyse.

17. Utilisation d'au moins un amide substitué de l'acide 5-aminométhyl-1H-pyrrole-2-carboxylique selon l'une quelconque des revendications 1 à 8 pour la préparation d'un médicament destiné au traitement de troubles de la mémoire.

18. Utilisation d'au moins un amide substitué de l'acide 5-aminométhyl-1H-pyrrole-2-carboxylique selon l'une quelconque des revendications 1 à 8 pour la préparation d'un médicament destiné au traitement de maladies de neurodégénérescence, de préférence la maladie de Parkinson, de Huntington et/ou d'Alzheimer.

19. Utilisation d'au moins un amide substitué de l'acide 5-aminométhyl-1H-pyrrole-2-carboxylique selon l'une quelconque des revendications 1 à 8 pour la préparation d'un médicament destiné au traitement de l'épilepsie.

20. Utilisation d'au moins un amide substitué de l'acide 5-aminométhyl-1H-pyrrole-2-carboxylique selon l'une quelconque des revendications 1 à 8 pour la préparation d'un médicament destiné à la diurèse.

21. Utilisation d'au moins un amide substitué de l'acide 5-aminométhyl-1H-pyrrole-2-carboxylique selon l'une quelconque des revendications 1 à 8 pour la préparation d'un médicament destiné au traitement de troubles cardiovasculaires.

22. Utilisation d'au moins un amide substitué de l'acide 5-aminométhyl-1H-pyrrole-2-carboxylique selon l'une quelconque des revendications 1 à 8 pour la préparation d'un médicament destiné au traitement de maladies de rétention d'eau.

23. Utilisation d'au moins un amide substitué de l'acide 5-aminométhyl-1H-pyrrole-2-carboxylique selon l'une quelconque des revendications 1 à 8 pour la préparation d'un médicament destiné au traitement de la motilité intestinale (diarrhée).

24. Utilisation d'au moins un amide substitué de l'acide 5-aminométhyl-1H-pyrrole-2-carboxylique selon l'une quelconque des revendications 1 à 8 pour la préparation d'un médicament destiné à supprimer le réflexe de miction.

25. Utilisation d'au moins un amide substitué de l'acide 5-aminométhyl-1H-pyrrole-2-carboxylique selon l'une quelconque des revendications 1 à 8 pour la préparation d'un médicament destiné au traitement de l'incontinence urinaire.

26. Utilisation d'au moins un amide substitué de l'acide 5-aminométhyl-1H-pyrrole-2-carboxylique selon l'une quelconque des revendications 1 à 8 pour la préparation d'un médicament destiné au traitement de l'anorexie.

27. Utilisation d'au moins un amide substitué de l'acide 5-aminométhyl-1H-pyrrole-2-carboxylique selon l'une quelconque des revendications 1 à 8 pour la préparation d'un médicament destiné au traitement des acouphènes.

28. Utilisation d'au moins un amide substitué de l'acide 5-aminométhyl-1H-pyrrole-2-carboxylique selon l'une quelconque des revendications 1 à 8 pour la préparation d'un médicament destiné au traitement du prurit.

29. Utilisation d'au moins un amide substitué de l'acide 5-aminométhyl-1H-pyrrole-2-carboxylique selon l'une quelconque des revendications 1 à 8 pour la préparation d'un médicament destiné au traitement de dépressions.

30. Utilisation d'au moins un amide substitué de l'acide 5-aminométhyl-1H-pyrrole-2-carboxylique selon l'une quelconque des revendications 1 à 8 pour la préparation d'un médicament destiné à la régulation, de préférence la stimulation de la prise d'aliments.

31. Utilisation d'au moins un amide substitué de l'acide 5-aminométhyl-1H-pyrrole-2-carboxylique selon l'une quelconque des revendications 1 à 8 pour la préparation d'un médicament destiné à la réduction du potentiel de dépendance d'opioïdes, de préférence de la morphine.

32. Utilisation d'au moins un amide substitué de l'acide 5-aminométhyl-1H-pyrrole-2-carboxylique selon l'une quelconque des revendications 1 à 8 pour la préparation d'un médicament destiné à la modulation de l'activité mobile.

33. Utilisation d'au moins un amide substitué de l'acide 5-aminométhyl-1H-pyrrole-2-carboxylique selon l'une quelconque des revendications 1 à 8 pour la préparation d'un médicament destiné à influencer le système cardiovasculaire, de préférence pour la vasodilatation des artères.

34. Utilisation d'au moins un amide substitué de l'acide 5-aminométhyl-1H-pyrrole-2-carboxylique selon l'une quelconque des revendications 1 à 8 pour la préparation d'un médicament destiné au traitement de dysfonctionnements sexuels, de préférence le dysfonctionnement érectile.

35. Utilisation d'au moins un amide substitué de l'acide 5-aminométhyl-1H-pyrrole-2-carboxylique selon l'une quelconque des revendications 1 à 8 pour la préparation d'un médicament destiné au traitement des maladies des voies respiratoires.

36. Utilisation d'au moins un amide substitué de l'acide 5-aminométhyl-1H-pyrrole-2-carboxylique selon l'une quelconque des revendications 1 à 8 pour la préparation d'un médicament destiné à la régulation de la gestion des électrolytes.
